# EUROPÄISCHE PATENTSCHRIFT

(11) **EP 1 274 986 B1**
(45) Veröffentlichungstag und Bekanntmachung des Hinweises auf die Patenterteilung: **13.07.2011**
(21) Anmeldenummer: 00956385.9
(22) Anmeldetag: 03.08.2000
(51) Int. Cl.: G01N 21/77, G01N 21/76, G01N 21/64, G01N 21/55, G01N 33/543

(54) **VORRICHTUNG UND VERFAHREN ZUR MULTIANALYTBESTIMMUNG**
DEVICE AND METHOD FOR DETERMINING MULTIPLE ANALYTES
DISPOSITIF ET PROCEDE DE DETERMINATION DE PLUSIEURS SUBSTANCES A ANALYSER

(30) Priorität: 13.08.1999 CH 148699
(43) Veröffentlichungstag der Anmeldung: 15.01.2003
(73) Patentinhaber: Bayer Technology Services GmbH, 51368 Leverkusen (DE)
(72) Erfinder: ABEL, Andreas, P., CH-4054 Basel (CH); DUBENECK, Gert, L., D-79189 Bad Krozingen (DE); EHRAT, Markus, CH-4312 Magden (CH); KRESBACH, Gerhard, M., D-79219 Staufen (DE); PAWLAK, Michael, D-79725 Laufenburg (DE); SCHÜRMANN-MADER, Eveline, CH-5089 Zeihen (CH)
(86) Internationale Anmeldenummer: PCT/EP2000/007529
(87) Internationale Veröffentlichungsnummer: WO 2001/013096

(56) Entgegenhaltungen:
- EP-A- 0 872 735
- WO-A-93/25892
- WO-A-94/27137
- WO-A-96/35940
- WO-A-97/35203
- WO-A-97/46707
- WO-A-98/09156
- WO-A-99/36766
- US-A- 5 459 325
- US-A- 5 747 274

## Beschreibung

Die Erfindung betrifft eine Vorrichtung, umfassend eine Vielzahl von Probenbehälmissen, in denen in jeweils fünf oder mehr diskreten Messbereichen unterschiedliche biochemische oder biologische Erkennungselemente zur spezifischen Erkennung und Bindung unterschiedlicher Analyten immobilisert sind, wobei die biologischen oder biochemischen Erkennungselemente in diesen Messbereichen entweder direkt oder über eine Haftvermittlungsschicht und gegebenenfalls noch weitere Schichten auf der Oberfläche eines optischen Wellenleiters als Bestandteil einer Sensorplattform immobilisiert sind, welche eine Begrenzungsfläche dieser Probenbehältnisse bildet. Dabei kann die Sensorplattform in verschiedenen Ausführungsformen vorliegen.

Die Erfindung betrifft auch ein analytisches System, welches eine erfindungsgemässe Vorrichtung mit verschiedenen Ausführungsformen der Sensorplattform, ein optisches System zur Lumineszenzbestimmung mit mindestens einer Anregungslichtquelle und mindestens einem Detektor zur Erfassung des von den Messbereichen ausgehenden Lichts sowie Zuführungsmittel, um eine oder mehrere Proben mit den Messbereichen auf der Sensorplattform in Kontakt zu bringen, umfasst. Weiterer Gegenstand der Erfindung sind Lumineszenznachweisverfahren mit erfindungsgemässen Sensorplattformen, optischen Systemen und analytischen Systemen sowie die Verwendung dieser Verfahren für die quantitative Affinitätssensorik sowie für verschiedene weitere Anwendungen.

Ziel der vorliegenden Erfindung ist die Bereitstellung von Messanordnungen mit einer Vielzahl von kleinvolumigen Probenbehältnissen, in denen eine Vielzahl von Analyten durch ein hochempfindliches Nachweisverfahren gleichzeitig bestimmt werden kann. Zur Bestimmung einer Vielzahl von Analyten sind gegenwärtig vor allem Verfahren verbreitet, in denen in sogenannten Mikrotiterplatten der Nachweis unterschiedlicher Analyten in diskreten Probenbehältnissen oder "Wells" dieser Platten erfolgt. Am weitesten verbreitet sind dabei Platten mit einem Raster von 8 x 12 Wells auf einer Grundfläche von typischerweise ca. 8 cm x 12 cm, wobei zur Füllung eines einzelnen Wells ein Volumen von einigen hundert Mikrolitern erforderlich ist. Für zahlreiche Anwendungen wäre es jedoch wünschenswert, mehrere Analyten in einem einzigen Probenbehältnis, unter Einsatz eines möglichst kleinen Probenvolumens zu bestimmen.

In der US-P 5,747,274 werden Messanordnungen und Verfahren zur Früherkennung eines Herzinfarkts, durch die Bestimmung mehrerer von mindestens drei Herzinfarktmarkern beschrieben, wobei die Bestimmung dieser Marker in individuellen oder in einem gemeinsamen Probenbehältnis erfolgen kann, wobei im letzteren Falle, der gegebenen Beschreibung folgend, ein einziges Probenbehältnis als ein durchgehender Flusskanal ausgebildet ist, dessen eine Begrenzungsfläche beispielsweise eine Membran bildet, auf der Antikörper für die drei verschiedenen Marker immobilisert sind. Es gibt jedoch keine Hinweise auf eine Bereitstellung von mehreren derartigen Probenbehältnissen oder Flusskanälen auf einem gemeinsamen Träger. Ausserdem werden keine geometrischen Angaben über die Grössen der Messflächen gegeben.

In den WO 84/01031, US-P 5,807,755, US-P 5,837,551 und US-P 5,432,099 wird die Immobilisierung für den Analyten spezifischer Erkennungselemente in Form kleiner "Spots" mit teilweise deutlich unter 1 mm² Fläche auf festen Trägem vorgeschlagen, um durch Bindung eines nur kleinen Teils vorhandener Analytmoleküle eine nur von der Inkubationszeit abhängige, aber - in Abwesenheit eines kontinuierlichen Flusses - vom absoluten Probenvolumen im wesentlichen unabhängige Konzentrationsbestimmung des Analyten vornehmen zu können. Die in den zugehörigen Ausführungsbeispielen beschriebenen Messanordnungen beruhen auf Fluoreszenznachweisen in konventionellen Mikrotiterplatten. Dabei werden auch Anordnungen beschrieben, in denen Spots von bis zu drei unterschiedlichen fluöreszenzmarkierten Antikörpern in einem gemeinsamen Mikrotiterplattenwell ausgemessen werden. Den in diesen Patentschriften dargelegten theoretischen Überlegungen folgend, wäre eine Minimierung der Spotgrösse wünschenswert. Limitierend wirke jedoch die minimale Signalhöhe, die vom Untergrundsignal unterschieden werden könne.

Zur Erreichung tieferer Nachweisgrenzen sind in den vergangenen Jahren zahlreiche Messanordnungen entwickelt worden, in denen der Nachweis des Analyten auf dessen Wechselwirkung mit dem evaneszenten Feld beruht, welches mit der Lichtleitung in einem optischen Wellenleiter verbunden ist, wobei auf der Oberfläche des Wellenleiters biochemische oder biologische Erkennungselemente zur spezifischen Erkennung und Bindung der Analytmoleküle immobilisiert sind.

Koppelt man eine Lichtwelle in einen optischen Wellenleiter ein, der von optisch dünneren Medien, d.h. Medien mit niedrigerem Brechungsindex umgeben ist, so wird sie durch Totalreflektion an den Grenzflächen der wellenleitenden Schicht geführt. In die optisch dünneren Medien tritt dabei ein Bruchteil der elektromagnetischen Energie ein. Diesen Anteil bezeichnet man als evaneszentes oder quergedämpftes Feld. Die Stärke des evaneszenten Feldes ist sehr stark abhängig von der Dicke der wellenleitenden Schicht selbst sowie vom Verhältnis der Brechungsindices der wellenleitenden Schicht und der sie umgebenden Medien. Bei dünnen Wellenleitern, d. h. Schichtdicken von derselben oder niedrigerer Dicke als der zu führenden Wellenlänge, können diskrete Moden des geleiteten Lichts unterschieden werden. Derartige Verfahren haben den Vorteil, dass die Wechselwirkung mit dem Analyten auf die Eindringtiefe des evaneszenten Feldes ins angrenzende Medium, in der Grössenordnung von einigen hundert Nanometern, beschränkt ist und Störsignale aus der Tiefe des Mediums weitgehend vermieden werden können. Die ersten vorgeschlagenen derartigen Messanordnungen beruhten auf hochmultimodalen, selbsttragenden Einschichtwellenleitem, wie beispielsweise Fasern oder Plättchen aus transparentem Kunststoff oder Glas, mit Stärken von einigen hundert Mikrometern bis zu mehreren Millimetern.

In der US-P 4,978,503 wird eine Messanordnung beschrieben, in der eine flüssige Probe über Kapillarkräfte in eine Kavität eingezogen wird, wobei eine optisch transparente Seitenwand als selbsttragender Multimode-Wellenleiter ausgebildet ist, wobei zumindest auf einem Teil seiner der Kavität zugewandten Oberfläche ("Patch") ein biochemisches Erkennungselement zur Erkennung und Bindung eines Analyten aus einer Probe immobilisiert ist. Zwar wird in der obigen Patentschrift die Herstellung von Arrays solcher Anordnungen mit mehreren diskreten Patches beschrieben, jedoch geht aus den weiteren Erläuterungen hervor, dass die Patches seitlich jeweils durch Verbindungsstege, vorgesehen zur Verbindung der Boden- und der Deckelplatten der Kapillaren, voneinander abgegrenzt sind, so dass nach Vereinzelung der Arrays jede Kapillare nur einen Patch immobilisierter Erkennungselemente enthält. Weiterhin ist in dieser Anordnung von Nachteil, dass ein Austausch der in die Kapillare gelangten Flüssigkeit nicht vorgesehen ist, welches beispielsweise in einem mehrstufigen Assay wünschenswert wäre.

In der WO 94/27137 werden Messanordnungen beschrieben, in denen "Patches" mit unterschiedlichen Erkennungselementen, zum Nachweis unterschiedlicher Analyten, auf einem selbstragenden optischen Substratwellenleiter (Einschichtwellenleiter) mit Stirnflächenlichteinkopplung immobilisiert sind, wobei die räumlich selektive Immobilisierung mittels photoaktivierbarer Crosslinker erfolgt. Gemäss der gegebenen Beschreibung können mehrere Patches in Reihe in gemeinsamen parallelen Flusskanälen oder Probenbehältnissen angeordnet sein, wobei sich die parallelen Flusskanäle oder Probenbehältnisse über die gesamte Länge des als Sensor genutzten Bereichs des Wellenleiters erstrecken, um eine Beeinträchtigung der Lichtleitung im Wellenleiter zu vermeiden. Hinweise auf eine zweidimensionale Integration einer Vielzahl von Patches in Probenbehältnissen relativ kleiner Ausmasse, d.h. von deutlich weniger als 1cm² Grundfläche, werden jedoch nicht gegeben. In einer ähnlichen Anordnung werden in der WO 97/35203 verschiedene Ausführungsformen einer Anordnung beschrieben, in der in parallelen, separaten Flusskanälen oder Probenbehältnissen für die Probe und Kalibrationslösungen niedriger und gegebenenfalls zusätzlich hoher Analytkonzentration unterschiedliche Erkennungselemente zur Bestimmung verschiedener Analyten jeweils immobilisiert sind. Auch hier wird jedoch keinerlei Hinweis gegeben, wie eine hohe Integrationsdichte unterschiedlicher Erkennungselemente in einer zu einem gemeinsamen Behältnis zugeführten Probe erreicht werden könnte. Ausserdem ist die Emfindlichkeit hoch multimodaler, selbstragender Einschichtwellenleiter für eine Vielzahl von Anwendungen, in denen die Erreichung sehr tiefer Nachweisgrenzen erforderlich ist, nicht ausreichend.

Zur Verbesserung der Empfindlichkeit und gleichzeitig einfacheren Herstellung in Massenfabrikation wurden planare Dünnschichtwellenleiter vorgeschlagen. Ein planarer Dünnschichtwellenleiter besteht im einfachsten Fall aus einem Dreischichtsystem: Trägermaterial, wellenleitende Schicht, Superstrat (bzw. zu untersuchende Probe), wobei die wellenleitende Schicht den höchsten Brechungsindex besitzt. Zusätzliche Zwischenschichten können die Wirkung des planaren Wellenleiters noch verbessern.

Es sind verschiedene Verfahren für die Einkopplung von Anregungslicht in einen planaren Wellenleiter bekannt. Die am frühesten benutzten Verfahren beruhten auf Stirnflächenkopplung oder Prismenkopplung, wobei zur Verminderung von Reflexionen infolge von Luftspalten im allgemeinen eine Flüssigkeit zwischen Prisma und Wellenleiter aufgebracht wird. Diese beiden Methoden sind vor allem in Verbindung mit Wellenleitern relativ grosser Schichtdicke, d. h. insbesondere selbsttragenden Wellenleitern, sowie bei einem Brechungsindex des Wellenleiters von deutlich unter 2 geeignet. Zur Einkopplung von Anregungslicht in sehr dünne, hochbrechende wellenleitende Schichten ist demgegenüber die Verwendung von Koppelgittern eine wesentlich elegantere Methode.

Es können verschiedene Methoden zum Analytnachweis im evaneszenten Feld geführter Lichwellen in optischen Schichtwellenleitern unterschieden werden. Aufgrund des eingesetzten Messprinzips kann man beispielsweise zwischen Fluoreszenz- oder allgemeiner Lumineszenzmethoden auf der einen Seite und refraktiven Methoden andererseits unterscheiden. Hierbei können Verfahren zur Erzeugung einer Oberflächenplasmonenresonanz in einer dünnen Metallschicht auf einer dielektrischen Schicht mit niedrigerem Brechungsindex in die Gruppe der refraktiven Methoden mit einbezogen werden, sofern als Basis zur Bestimmung der Messgrösse der Resonanzwinkel des eingestrahlten Anregungslichts zur Erzeugung der Oberflächenplasmonenresonanz dient. Die Oberflächenplasmonenresonanz kann aber auch zur Verstärkung einer Lumineszenz oder zur Verbesserung des Signal-zu-Hintergrund-Verhältnisses in einer Lumineszenzmessung verwendet werden. Die Bedingungen zur Erzeugung einer Oberflächenplasmonenresonanz sowie zur Kombination mit Lumineszenzmessungen sowie mit wellenleitenden Strukturen sind vielfach in der Literatur beschrieben, beispielsweise in den US-Patenten US-P 5,478,755, US-P 5,841,143, US-P 5,006,716 und US-P 4,649,280.

Mit dem Begriff "Lumineszenz" wird in dieser Anmeldung die spontane Emission von Photonen im ultravioletten bis infraroten Bereich nach optischer oder nichtoptischer, wie beispielsweise elektrischer oder chemischer oder biochemischer oder thermischer Anregung, bezeichnet. Beispielsweise sind Chemilumineszenz, Biolumineszenz, Elektrolumineszenz und insbesondere Fluoreszenz und Phosphoreszenz unter dem Begriff "Lumineszenz" mit eingeschlossen.

Bei den refraktiven Messmethoden wird die Änderung des sogenannten effektiven Brechungsindex aufgrund molekularer Adsorption oder Desorption auf dem Wellenleiter zum Nachweis des Analyten benutzt. Diese Änderung des effektiven Brechungsindex wird, im Falle von Gitterkoppler-Sensoren, bestimmt aus der Änderung des Koppelwinkels für die Ein- oder Auskopplung von Licht in oder aus dem Gitterkoppler-Sensor, und im Falle von interferometrischen Sensoren aus der Änderung der Phasendifferenz zwischen dem in einem Sensorarm und einem Referenzarm des Interferometers geführten Messlichts.

Der Stand der Technik zum Einsatz von einem oder mehreren Koppelgittern zum Ein- und/oder Auskoppeln geführter Wellen mittels eines oder mehrerer Koppelgitter ist beispielsweise in K. Tiefenthaler, W. Lukosz,"Sensitivity of grating couplers as integrated-optical chemical sensors", J. Opt. Soc. Am. B6, 209 (1989); W. Lukosz, Ph.M. Nellen, Ch. Stamm, P. Weiss, "Output Grating Couplers on Planar Waveguides as Integrated, Optical Chemical Sensors", Sensors and Actuators B1, 585 (1990), und in T. Tamir, S.T. Peng, "Analysis and Design of Grating Couplers", Appl. Phys. 14, 235-254 (1977), beschrieben.

In der US-P 5,738,825 wird eine Anordnung bestehend aus einer Mikrotiterplatte mit vollständig durchgehenden Bohrungen und einem Dünnschichtwellenleiter als Bodenplatte, letzterer bestehend aus einem dünnen wellenleitenden Film auf einem transparenten, selbsttragenden Substrat, beschrieben. In Kontakt mit den aus der durchbohrten Mikrotiterplattte und dem Dünnschichtwellenleiter als Bodenplatte gebildeten, offenen Probenbehältnissen sind jeweils Beugungsgitter für die Ein- und Auskopplung von Anregungslicht vorgesehen, um aus Änderungen des beobachteten Koppelwinkels die dafür verantwortlichen Änderungen des effektiven Brechungsindex infolge von Adsorption oder Desorption von nachzuweisenden Analytmolekülen zu bestimmen. Ein Nachweis mehrerer Analyten in einem Probenbehältnis, durch Bindung an verschiedene auf der Gitterstruktur in dem Probenbehältnis immobilisierte Erkennungselemente, ist jedoch nicht vorgesehen und erscheint aufgrund des refraktiven Messprinzips, d. h. der Bestimmung von relativen Änderungen des Koppelwinkels, auch schwer realisierbar.

Die genannten refraktiven Methoden haben den Vorteil, dass sie ohne Verwendung zusätzlicher Markierungsmoleküle, sogenannter molekularer Labels, eingesetzt werden können. Der Nachteil dieser labelfreien Methoden ist jedoch, dass die damit erzielbaren Nachweisgrenzen aufgrund der geringen Selektivität des Messprinzips, in Abhängigkeit von dem Molekulargewicht des Analyten auf pico- bis nanomolare Konzentrationsbereiche beschränkt sind, was für viele Anwendungen der modernen Spurenanalytik, beispielsweise für diagnostische Applikationen, nicht ausreichend ist.

Zur Erreichung tieferer Nachweisgrenzen erscheinen lumineszenzbasierende Methoden aufgrund grösserer Selektivität der Signalerzeugung besser geeignet. Dabei ist die Lumineszenzanregung auf die Eindringtiefe des evaneszenten Feldes in das optisch dünnere Medium, also auf die unmittelbare Umgebung des wellenleitenden Bereichs mit einer Eindringtiefe in der Grössenordnung von einigen hundert Nanometern ins Medium beschränkt. Dieses Prinzip wird evaneszente Lumineszenzanregung genannt.

Mittels hochbrechender Dünnschichtwellenleiter, in Kombination mit Lumineszenzdetektion, basierend auf einem nur einige hundert Nanometer dünnen wellenleitenden Film auf einem transparenten Trägermaterial, konnte in den letzten Jahren die Empfindlichkeit deutlich gesteigert werden. Beispielsweise wird in der WO 95/33197 eine Methode beschrieben, in der das Anregungslicht über ein Reliefgitter als diffraktives optisches Element in den wellenleitenden Film eingekoppelt wird. Die Oberfläche der Sensorplattform wird mit einer den Analyten enthaltenden Probe in Kontakt gebracht, und die isotrop angestrahlte Lumineszenz in der Eindringtiefe des evaneszenten Feldes befindlicher lumineszenzfähiger Substanzen wird mittels geeigneter Messvorrichtungen, wie zum Beispiel Photodioden, Photomultiplier oder CCD-Kameras, gemessen. Es ist auch möglich, den in den Wellenleiter rückgekoppelten Anteil der evaneszent angeregten Strahlung über ein diffraktives optisches Element, zum Beispiel ein Gitter, auszukoppeln und zu messen. Diese Methode ist zum Beispiel in der WO 95/33198 beschrieben.

Ein Nachteil aller oben im Stand der Technik, insbesondere in der WO 95/33197 und der WO 95/33198, beschriebenen Verfahren zur Detektion evaneszent angeregter Lumineszenz mit Dünnschichtwellenleitern liegt jedoch darin, dass auf der als homogener Film ausgebildeten Sensorplattform jeweils nur eine Probe analysiert werden kann. Um weitere Messungen auf derselben Sensorplattform durchführen zu können, sind jedesmal aufwendige Wasch- bzw. Reinigungsschritte notwendig. Dies gilt insbesonders, wenn ein von der ersten Messung verschiedener Analyt detektiert werden soll. Im Falle eines Immunoassays bedeutet dies im allgemeinen, dass die gesamte immobilisierte Schicht auf der Sensorplattform ausgetauscht oder gleich eine neue Sensorplattform als ganzes verwendet werden muss.

Zur gleichzeitigen oder aufeinanderfolgenden Durchführung von ausschliesslich lumineszenzbasierenden Mehrfachmessungen mit im wesentlichen monomodalen, planaren anorganischen Wellenleitern sind, z. B. in der WO 96/35940, Vorrichtungen (Arrays) bekannt geworden, in denen auf einer Sensorplattform wenigstens zwei getrennte wellenleitende Bereiche angeordnet sind, die getrennt mit Anregungslicht beaufschlagt werden. Die Aufteilung der Sensorplattform in getrennte wellenleitende Bereiche hat allerdings nachteilig zur Folge, dass der Platzbedarf für diskrete Messbereiche, in diskreten wellenleitenden Bereichen auf der gemeinsamen Sensorplattform relativ gross ist und daher nur eine verhältnismässige geringe Dichte unterschiedlicher Messbereiche (oder sogenannter "features") erreicht werden kann.

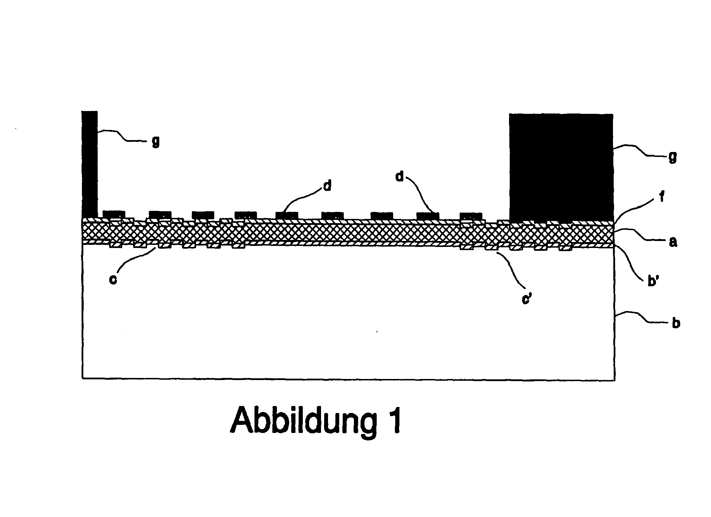

Im Sinne der vorliegenden Erfindung sollen räumlich getrennte Messbereiche (d) (gemäss Abbildung 1) durch die geschlossene Fläche definiert werden, die dort immobilisierte biologische oder biochemische oder synthetische Erkennungselementen zur Erkennung eines Analyten aus einer flüssigen Probe einnehmen. Diese Flächen können dabei eine beliebige Geometrie, beispielsweise die Form von Punkten, Kreisen, Rechtecken, Dreiecken, Ellipsen oder Linien, haben.

In der WO 98/22799 werden neben einer Vielzahl weiterer Vorrichtungen für die Ausgestaltung von Probenbehältnissen für Messanordnungen zur Bestimmung der im evaneszenten Feld eines planaren Wellenleiters angeregten Lumineszenz auch solche Vorrichtungen vorgeschlagen, welche der Form bekannter Mikrotiterplatten entsprechen. Die Bestimmung mehrerer Analyten durch Bindung an verschiedene innerhalb eines einzelnen Probenbehältnisse immobiliserte Erkennungelemente ist jedoch auch hier nicht vorgesehen.

Es besteht daher ein Bedürfnis für die Bereitstellung von kleinvolumigen Probenbehältnissen in Kombination mit geeigneten Sensorplattformen, in denen eine Vielzahl von Analyten durch ein hochempfindliches Nachweisverfahren gleichzeitig ohne grossen Aufwand bestimmt werden kann.

Es wurde nun überraschend gefunden, dass durch die gewählte Kombination der hocheffizienten Fluoreszenzanregung im evaneszenten Feld eines geeigneten optischen Wellenleiters, insbesondere von Dünnschichtwellenleitern, und Anordnung von mehreren Messbereichen mit unterschiedlichen biologischen oder biochemischen Erkennungselementen in einem einzigen Probenbehältnis, als Teil eines zweidimensionalen Arrays von Probenbehältnissen auf einer gemeinsamen Sensorplattform, wesentlich tiefere Nachweisgrenzen und höhere Integrationsdichten von Messbereichen erzielt werden können als mit den bekannten konventionellen Vorrichtungen und Verfahren (z. B. ELISA-Tests auf klassischen Mikrotiterplatten). Dabei kann im jeweiligen Nachweisschritt des Verfahrens, unter Zugabe von lumineszenzmarkierten Bindungspartnern des jeweiligen Bioaffinitätsassays, der Lumineszenznachweis ohne zusätzlichen Waschschritt durchgeführt werden, was eine Vereinfachung des Verfahrens bedeutet.

Überraschend kann durch die Anordnung mehrerer Messbereiche und unterschiedlicher biologischer oder biochemischer Erkennungselemente in einem einzigen Probenbehältnis, unter Zugabe einer einzelnen Probe, in einer Einzelmessung bereits statistisch relevante Information, beispielsweise zur Assayreproduzierbarkeit, generiert werden. Zugleich zeigen die dabei erzeugten Daten sogar eine geringere Variabilität als die Ergebnisse einer entsprechenden Anzahl von Einzelmessungen in diskreten Probenbehältnissen, da mit der Simultanmessung eine Vielzahl möglicher zusätzlicher Variabilitäts- und Fehlerquellen für die voneinander unabhängigen Einzelmessungen ausgeschlossen werden. Damit kann eine bedeutende Einsparung an Analysenzeit und einzusetzender Proben- und Reagentienmenge im Vergleich zu den vorher beschriebenen und bekannten Nachweisverfahren (z. B. ELISA-Tests) erzielt werden. Insbesondere ist es auch möglich, Referenzierungsmessungen, insbesondere zur Bestimmung chemischer Parameter wie z.B. unspezifische Bindung, Crossreaktivitäten, Bestimmung von pH oder pO₂ der Probenlösung etc., in jedem einzelnen Probenbehältnis durchzuführen, indem einer oder mehrere der Messbereiche in einem Probenbehältnis für derartige Referenzierungsmessungen verwendet werden. Damit kann einerseits die Zuverlässigkeit dieser Referenzierungsmessungen deutlich erhöht werden gegenüber den herkömmlichen Verfahren, in denen diese Messungen in jeweils verschiedenen Probenbehältnissen vorgenommen wurden.

Weiterhin kann mit der Vorrichtung und dem damit durchgeführten Nachweisverfahren die Anzahl der Messungen pro Platte und damit der Durchsatz auf ein Vielfaches erhöht werden. Damit können die Reproduzierbarkeit der Messungen und Vergleichbarkeit der Ergebnisse mit verschiedenen Proben deutlich verbessert werden, u. a. weil die üblichen starken Platte-zu-Platte Schwankungen wegfallen. Mit beispielsweise 96 Probenbehältnissen, in denen sich jeweils 400 Messbereiche befinden, auf einer einzigen Sensorplattform ist es möglich, umfangreiche Messreihen, beispielsweise für die pharmazeutische Produktentwicklung, auf einer einzigen erfindungsgemässen Vorrichtung durchzuführen, wofür typischerweise 400 Mikrotiterplatten benötigt wurden. Neben der deutlichen Verringerung von Proben- und Reagentienmaterial und einem damit vermindertem Aufwand für die Probenvorbereitung wird zugleich die Zuverlässigkeit und Genauigkeit der Ergebnisse aus den genannten Gründen deutlich erhöht.

Die erfindungsgemässe Vorrichtung und das erfindungsgemässe Verfahren bieten damit Möglichkeiten, die mit den herkömmlichen, bekannten Analysesystemen und -verfahren nicht bereitgestellt werden können:
- Schnelle Assayentwicklung auf einer einzigen Sensorplattform / Vorrichtung
- Bestimmung von Konzentrationskurven, bindungsspezifischem Crosstalk und Probenanalyse auf einer einzigen Sensorplattform / Vorrichtung
- Hochempfindliche Multianalytbestimmung in einer einzigen kleinvolumigen Probe
- Durchführung umfangreicher Messreihen auf einer einzigen Sensorplattform

Gegenstand der Erfindung ist eine Vorrichtung entsprechen Anspruch 1.

Von den jeweils fünf oder mehr Messbereichen in einem einzelnen Probenbehältnis können jeweils ein oder mehr Messbereiche zur Referenzierung verwendet werden.

Vorteilhaft werden Messbereiche zur Referenzierung gleicher chemischer oder optischer Parameter in mehreren über die Sensorplattform verteilten Probenbehältnissen verwendet, so dass die örtliche Verteilung besagter chemischer oder optischer Parameter auf der Sensorplattform bestimmt werden kann. Als zu bestimmender optischer Parameter ist dabei beispielsweise die Intensität des lokal verfügbaren Anregungslichts zu verstehen. Insbesondere können aus der so bestimmten Verteilung besagter chemischer Parameter beispielsweise Unterschiede in Zusammensetzung, Konzentration oder Volumen verschiedener Proben, beispielsweise resultierend aus der Probenvorbereitung, die in unterschiedliche Probenbehältnisse gegeben werden, berücksichtigt werden.

Es wird eine solche Ausführungsform der Vorrichtung bevorzugt, bei der die besagten Messbereiche in optischer Wechselwirkung mit dem evaneszenten Feld von in dem planaren optischen Wellenleiter geführtem Anregungslicht stehen.

Der optische Wellenleiter als Bestandteil der Sensorplattform kann ein multimodaler oder monomodaler Wellenleiter aus einem zumindest bei der Anregungs- und der Lumineszenzwellenlänge transparentem anorganischem Material, vorzugsweise Glas, oder organischem Material, vorzugsweise Kunststoff, besonders vorzugsweise ausgewählt aus der Gruppe, die von Polymethylmetacrylat, Polycarbonat, Polystyrol gebildet wird, sein. Insbesondere kann dann der optische Wellenleiter als Bestandteil der Sensorplattform selbsttragend sein.

Bevorzugt wird eine Vorrichtung, in welcher der optische Wellenleiter als Bestandteil der Sensorplattform ein optischen Schichtwellenleiter mit einer ersten optisch transparenten Schicht (a) (gemäss Abbildung 1) auf einer zweiten optisch transparenten Schicht (b) (gemäss Abbildung 1) mit niedrigerem Brechungsindex als Schicht (a) ist.

Das Material der zweiten optisch transparenten Schicht (b) kann aus Glas, Quarz oder einem transparenten thermoplastischer Kunststoff, bevorzugt aus der Gruppe, die von Polycarbonat, Polyimid, Polymethylmethacrylat oder Polystyrol gebildet wird, bestehen.

Zur Erzeugung eines möglichst starken evaneszenten Feldes an der Oberfläche der optisch transparenten Sicht (a) ist es wünschenswert, dass der Brechungsindex der wellenleitenden, optisch transparenten Schicht (a) wesentlich grösser als der Brechungsindex der benachbarten Schichten ist. Insbesondere ist es von Vorteil, wenn der Brechungsindex der ersten optisch transparenten Schicht (a) grösser als 1.8 ist.

Beispielsweise kann die erste optisch transparente Schicht (a) aus TiO₂, ZnO, Nb₂O₅, Ta₂O₅, HfO₂, oder ZrO₂ bestehen. Besonders bevorzugt wird, wenn die erste transparente optische Schicht (a) aus Ta₂O₅ oder Nb₂O₅ besteht.

Neben dem Brechungsindex der wellenleitenden optisch transparenten Schicht (a) ist deren Dicke der zweite massgebliche Parameter zur Erzeugung eines möglichst starken evaneszenten Feldes an deren Grenzflächen zu benachbarten Schichten mit niedrigerem Brechungsindex. Dabei nimmt die Stärke des evaneszenten Feldes mit abnehmender Dicke der wellenleitenden Schicht (a) zu, solange die Schichtdicke ausreicht, um mindestens einen Mode der Anregungswellenlänge zu führen. Dabei ist die minimale "Cut-off'-Schichtdicke zur Führung eines Modes abhängig von der Wellenlänge dieses Modes. Sie ist für längerwelliges Licht grösser als für kurzwelliges Licht. Mit Annäherung an die "Cut-off'-Schichtdicke nehmen allerdings auch ungewünschte Ausbreitungsverluste stark zu, was die Auswahl der bevorzugten Schichtdicke zusätzlich nach unten begrenzt. Bevorzugt sind solche Schichtdicken der optisch transparenten Schicht (a), welche nur die Führung von 1 bis 3 Moden einer vorgegebenen Anregungswellenlänge ermöglichen, ganz besonders bevorzugt sind Schichtdicken, welche in monomodalen Wellenleitern für diese Anregungswellenlänge führen. Dabei ist klar, dass sich der diskrete Modencharakter des geführten Lichts nur auf die transversalen Moden bezieht.

Diese Anforderungen führen dazu, dass vorteilhaft die Dicke der ersten optisch transparenten Schicht (a) 40 bis 300 nm beträgt. Ganz besonders vorteilhaft beträgt die Dicke der ersten optisch transparenten Schicht (a) 100 bis 200 nm.

Die Höhe der Ausbreitungsverluste eines in einer optisch wellenleitenden Schicht (a) geführten Modes wird in hohem Masse von der Oberflächenrauhigkeit einer darunter liegenden Trägerschicht sowie von Absorption durch möglicherweise in dieser Trägerschicht vorhandene Chromophoren bestimmt, was zusätzlich das Risiko der Anregung unerwünschter Lumineszenz in dieser Trägerschicht, durch Eindringen des evaneszenten Feldes des in der Schicht (a) geführten Modes, in sich birgt. Weiterhin kann es zum Auftreten thermischer Spannungen infolge unterschiedlicher thermischer Ausdehnungskoeffizienten der optisch transparenten Schichten (a) und (b) kommen. Im Falle einer chemisch empfindlichen optisch transparenten Schicht (b), sofern sie beispielsweise aus einem transparenten thermoplastischen Kunststoff besteht, ist es wünschenswert, ein Eindringen von Lösungsmitteln, welche die Schicht (b) angreifen könnten, durch eventuell in der optisch transparenten Schicht (a) vorhandene Mikroporen zu verhindern.

Daher ist es von Vorteil, wenn sich zwischen den optisch transparenten Schichten (a) und (b) und in Kontakt mit Schicht (a) eine weitere optisch transparente Schicht (b') (gemäss Abbildung 1) mit niedrigerem Brechungsindex als dem der Schicht (a) und einer Stärke von 5 nm - 10 000 nm, vorzugsweise von 10 nm - 1000 nm, befindet. Die Zwischenschicht hat die Aufgabe einer Verringerung der Oberflächenrauhigkeit unter der Schicht (a) oder der Verminderung des Eindringens des evaneszenten Feldes von in Schicht (a) geführtem Licht in die eine oder mehrere darunter liegende Schichten oder einer Verbesserung der Haftung der Schicht (a) auf der einen oder mehreren darunter liegenden Schichten oder der Verminderung von thermisch hervorgerufenen Spannungen innerhalb der optischen Sensorplattform oder der chemischen Isolation der optisch transparenten Schicht (a) von darunter liegenden Schichten mittels Abdichten von Mikroporen in der Schicht (a) gegen darunter liegende Schichten.

Es gibt eine Vielzahl von Methoden zur Aufbringung der biologischen oder biochemischen oder synthetischen Erkennungselementen auf die optisch transparente Schicht (a). Beispielsweise kann dieses durch physikalische Adsorption oder durch elektrostatische Wechselwirkung erfolgen. Die Orientierung der Erkennungselemente ist dann im allgemeinen statistisch. Ausserdem besteht die Gefahr, dass bei unterschiedlicher Zusammensetzung der den Analyten enthaltenden Probe oder der im Nachweisverfahren eingesetzten Reagentien ein Teil der immobilisierten Erkennungselemente fortgespült wird. Daher kann es von Vorteil sein, wenn zur Immobilisierung biologischer oder biochemischer oder synthetischer Erkennungselemente auf der optisch transparenten Schicht (a) eine Haftvermittlungsschicht (f) (gemäss Abbildung 1) aufgebracht ist. Diese Haftvermittlungsschicht sollte ebenfalls optisch transparent sein. Insbesondere sollte die Haftvermittlungsschicht nicht über die Eindringtiefe des evaneszenten Feldes aus der wellenleitenden Schicht (a) in das darüber liegende Medium hinausragen. Daher sollte die Haftvermittlungsschicht (f) eine Stärke von weniger als 200 nm, vorzugsweise von weniger als 20 nm, haben. Sie kann sich beispielsweise aus chemischen Verbindungen aus den Gruppen Silane, Epoxide, "selbstorganisierte funktionalisierte Monoschichten" zusammensetzen.

Wie in der Definition der Messbereiche festgestellt, ist es möglich, durch räumlich selektive Aufbringung von biologischen oder biochemischen oder synthetischen Erkennungselementen auf der Sensorplattform räumlich getrennte Messbereiche (d) zu erzeugen. Im Kontakt mit einem lumineszenzfähigen Analyten oder eines mit dem Analyten um die Bindung an die immobilisierten Erkennungselemente konkurrierenden lumineszenzmarkierten Analogen des Analyten oder eines weiteren lumineszenzmarkierten Bindungspartners in einem mehrstufigen Assay werden diese lumineszenzfähigen Moleküle nur selektiv in den Messbereichen an die Oberfläche der Sensorplattform binden, welche durch die Flächen definiert werden, die von den immobilisierten Erkennungselementen eingenommen werden.

Zur Aufbringung der biologischen oder biochemischen oder synthetischen Erkennungselemente können eines oder mehrere Verfahren verwendet werden aus der Gruppe von Verfahren, die von "Ink jet spotting, mechanischem Spotting mittels Stift oder Feder, micro contact printing, fluidische Kontaktierung der Messbereiche mit den biologischen oder biochemischen oder synthetischen Erkennungselementen durch deren Zufuhr in parallelen oder gekreuzten Mikrokanälen, unter Einwirkung von Druckunterschieden oder elektrischen oder elektromagnetischen Potentialen", gebildet werden.

Als biologische oder biochemische oder synthetische Erkennungselementen können Komponenten aus der Gruppe aufgebracht werden, die von Nukleinsäuren (DNA, RNA), Nukleinsäureanalogen (PNA), Antikörpern, Aptameren, membran-gebundenen und isolierten Rezeptoren, deren Liganden, Antigene für Antikörper, "Histidin-Tag-Komponenten", durch chemische Synthese erzeugte Kavitäten zur Aufnahme molekularer Imprints, etc. gebildet wird.

Unter der letztgenannten Art von Erkennungselementen sind Kavitäten zu verstehen, die in einem Verfahren hergestellt werden, welches als "molecular imprinting" in der Literatur beschrieben wurde. Dazu wird, meistens in organischer Lösung, der Analyt oder ein Analogon des Analyten, in einer Polymerenstruktur eingekapselt. Man bezeichnet ihn dann als "Imprint". Dann wird der Analyt oder sein Analogon unter Zugabe geeigneter Reagentien aus der Polymerenstruktur wieder herausgelöst, so dass er dort eine leere Kavität zurücklässt. Diese leere Kavität kann dann als eine Bindungsstelle mit hoher sterischer Selektivität in einem späteren Nachweisverfahren eingesetzt werden.

Es ist auch möglich, dass als biochemische oder biologische Erkennungselemente ganze Zellen oder Zellfragmente aufgebracht werden. In vielen Fällen wird die Nachweisgrenze eines analytischen Verfahrens limitiert durch Signale sogenannter unspezifischer Bindung, d. h. durch Signale, welche durch Bindung des Analyten oder anderer zum Nachweis des Analyten eingesetzter Verbindungen erzeugt werden, welche nicht nur im Bereich der eingesetzten immobilisierten biologischen oder biochemischen oder synthetischen Erkennungselementen, sondern auch in davon unbedeckten Bereichen einer Sensorplattform gebunden werden, beispielsweise durch hydrophobe Adsorption oder durch elektrostatische Wechselwirkungen. Daher ist es von Vorteil, wenn zwischen den räumlich getrennten Messbereichen (d) gegenüber dem Analyten "chemisch neutrale" Verbindungen zur Verminderung unspezifischer Bindung oder Adsorption aufgebracht sind. Als "chemisch neutrale" Verbindungen werden dabei solche Stoffe bezeichnet, welche selbst keine spezifischen Bindungsstellen zur Erkennung und Bindung des Analyten oder eines Analogen des Analyten oder eines weiteren Bindungspartners in einem mehrstufigen Assay aufweisen und durch ihre Anwesenheit den Zugang des Analyten oder seines Analogen oder der weiteren Bindungspartner zur Oberfläche der Sensorplattform blockieren.

Als "chemisch neutrale" Verbindungen können beispielsweise Stoffe aus den Gruppen eingesetzt werden, die zum Beispiel von Albuminen, insbesondere Rinderserumalbumin, Heringssperma oder auch von Polyethylenglycolen gebildet werden.

Es wird bevorzugt, dass die Einkopplung des Anregungslichts zu den Messbereichen (d) mithilfe von einer oder mehreren Gitterstrukturen (c) (gemäss Abbildung 1) erfolgt, die in der optisch transparenten Schicht (a) ausgeprägt sind.

Ein optisches Übersprechen von einem Messbereich zu anderen Messbereichen kann durch die Ausbreitung von in den Wellenleiter eingekoppeltem Licht, insbesondere auch von rückgekoppeltem Lumineszenzlicht, erfolgen. Zur Verhinderung eines solchen Übersprechens ist es von Vorteil, wenn Auskopplung von in der optisch transparenten Schicht (a) geführtem Licht mithilfe von Gitterstrukturen (c') (gemäss Abbildung 1) erfolgt, die in der optisch transparenten Schicht (a) ausgeprägt sind. Dabei können in der optisch transparenten Schicht (a) ausgeprägte Gitterstrukturen (c) und (c') gleiche oder unterschiedliche Periode haben und parallel oder nicht parallel zueinander ausgerichtet sein.

Insbesondere können Gitterstrukturen (c) und (c') wechselseitig als Ein- und / oder Auskoppelgitter verwendet werden.

Die Gitterstrukturen (c) und gegebenenfalls zusätzlich vorhandene Gitterstrukturen (c') weisen vorteilhaft eine Periode von 200 nm - 1000 nm auf, und die Modulationstiefe der Gitter beträgt vorteilhaft 3 bis 100 nm, bevorzugt 10 bis 30 nm.

Es wird bevorzugt, dass das Verhältnis von Modulationstiefe zur Dicke der ersten optisch transparenten Schicht (a) gleich oder kleiner als 0,2 ist.

Eine Gitterstruktur (c) kann ein Reliefgitter mit Rechteck-, Dreieck- oder halbkreisförmigem Profil oder ein Phasen- oder Volumengitter mit einer periodischen Modulation des Brechungsindex in der im wesentlichen planaren optisch transparenten Schicht (a) sein.

Zur Verstärkung einer Lumineszenz oder zur Verbesserung des Signal-zu-Hintergrund-Verhältnisses kann es weiterhin von Vorteil sein, wenn zwischen der optisch transparenten Schicht (a) und den immobilisierten biologischen oder biochemischen oder synthetischen Erkennungselementen eine dünne Metallschicht, vorzugsweise aus Gold oder Silber, gegebenenfalls auf einer zusätzlichen dielektrischen Schicht mit niedrigerem Brechungsindex als der Schicht (a), beispielsweise aus Silica oder Magnesiumfluorid, aufgebracht ist, wobei die Dicke der Metallschicht und der eventuellen weiteren Zwischenschicht so ausgewählt ist, dass ein Oberflächenplasmon bei der Anregungswellenlänge und / oder der Lumineszenzwellenlänge angeregt werden kann.

Für viele Anwendungen ist es von Vorteil, wenn die Gitterstruktur (c) ein diffraktives Gitter mit einer einheitlichen Periode ist. Der Resonanzwinkel zur Einkopplung des Anregungslichts über die Gitterstruktur (c) zu den Messbereichen ist dann im gesamten Bereich der Gitterstruktur einheitlich. Will man jedoch beispielsweise das Anregungslicht verschiedener Lichtquellen mit deutlich unterschiedlicher Wellenlänge einkoppeln, so können sich die entsprechenden Resonanzwinkel für die Einkopplung deutlich unterscheiden, was die Verwendung zusätzlicher Justierelemente in einem optischen System zur Aufnahme der Sensorplattform erforderlich machen kann oder zu räumlich sehr ungünstigen Koppelwinkeln führen kann. Beispielsweise zur Verringerung stark unterschiedlicher Koppelwinkel kann es daher von Vorteil sein, wenn die Gitterstruktur (c) ein multidiffraktives Gitter ist.

Für Anwendungen, in denen Änderungen von Ein- und Auskoppelwinkeln infolge eines Kontakts mit der Probe vermieden werden sollen, kann es vorteilhaft sein, wenn sich die Gitterstrukturen (c) und gegebenenfalls zusätzlich vorhandenen Gitterstrukturen (c') ausserhalb des Bereichs der Probenbehältnisse befinden.

Für andere Anwendungen, insbesondere bei grossflächiger Anregung wird demgegenüber bevorzugt, wenn sich Gitterstrukturen (c) und gegebenenfalls zusätzlich vorhandene Gitterstrukturen (c') über den Bereich mehrerer oder alle Probenbehältnisse erstrecken.

Soll über eine Gitterstruktur (c) eingekoppeltes Anregungslicht die optisch transparente Schicht (a) durchgehend über Bereiche durchlaufen, die sich über mehrere Probenbehältnisse erstrecken, so ist es notwendig, dass das Material der mit der Sensorplattform in Kontakt stehenden, dicht verschliessenden Schicht (g) an der Auflageoberfläche zu der Sensorplattform mindestens in der Eindringtiefe des evaneszenten Feldes sowohl für die Anregungsstrahlung als auch für die angeregte Lumineszenzstrahlung transparent ist.

Zur Verminderung von Reflexionen oder Streulicht ist es dann von Vorteil, wenn die Schicht (g) als zweilagige Schicht vorliegt, deren erste, welche mit der Oberfläche der Sensorplattform in Kontakt gebracht wird, sowohl für die Anregungsstrahlung als auch für die angeregte Lumineszenzstrahlung transparent ist, während die dann anschliessende, sich im weiteren Abstand von der Sensorplattform befindliche Schicht im Spektralbereich der Anregungsstrahlung und der angeregten Lumineszenzstrahlung absorbierend ist.

Für Anwendungen, in denen die Ausbreitung von Anregungslicht und gegebenenfalls erzeugtem und in die optisch transparente, wellenleitende Schicht (a) rückgekoppeltem Lumineszenzlicht in dieser Schicht jeweils auf den Bereich eines einzelnen Probenbehältnisses beschränkt werden soll, ist es demgegenüber von Vorteil, wenn das Material der mit der Sensorplattform in Kontakt stehenden, dicht verschliessenden Schicht (g) im Spektralbereich der Anregungsstrahlung und der angeregten Lumineszenzstrahlung absorbierend ist.

Vorteilhaft ist, wenn das Material der mit der Sensorplattform in Kontakt stehenden, dicht verschliessenden Schicht (g) auf der Sensorplattform selbsthaftend ist.

Daher wird insbesondere bevorzugt, dass das Material der mit der Sensorplattform in Kontakt stehenden, dicht verschliessenden Schicht (g) aus einem Polysiloxan besteht.

Es wird bevorzugt, dass sich in einem Probenbehältnis 5 - 1000, bevorzugt 10 - 400 Messbereiche befinden.

Weiterhin wird bevorzugt, dass ein einzelner Messbereich in einem Probenbehältnis eine Fläche von 0.001 - 6 mm² einnimmt, wobei die Messbereiche gleiche oder unterschiedliche Grösse haben.

Die Probenbehältnisse haben vorteilhaft jeweils ein Volumen von 100 nl - 1 ml.

Die erfindungsgemässe Vorrichtung kann sowohl als geschlossenes Fliesssystem als auch als offenes System betrieben werden. Im ersten Fall ist die Vorrichtung so gestaltet, dass die Probenbehältnisse auf der von der optisch transparenten Schicht (a) abgewandten Seite, mit Ausnahme von Ein- und / oder Auslassöffnungen für die Zufuhr oder den Auslass der Proben und gegebenenfalls zusätzlicher Reagentien, geschlossen sind und die Zufuhr oder der Auslass von Proben und gegebenenfalls zusätzlicher Reagentien in einem geschlossenen Durchflusssystem erfolgen, wobei im Falle der Flüssigkeitszufuhr zu mehreren Messbereichen oder Segmenten mit gemeinsamen Einlass- und Auslassöffnungen diese bevorzugt spalten- oder zeilenweise addressiert werden.

Dabei kann die Zufuhr der Proben und gegebenenfalls zusätzlichen Reagentien in parallelen oder gekreuzten Mikrokanälen, unter Einwirkung von Druckunterschieden oder elektrischen oder elektromagnetischen Potentialen erfolgen.

Im Falle eines offenen Systems ist die erfindungsgemässe Vorrichtung so gestaltet, dass die Probenbehältnisse auf der von der optisch transparenten Schicht (a) abgewandten Seite Öffnungen zur lokal addressierten Zugabe oder Entfernung der Proben oder anderer Reagentien besitzen. Zusätzlich können Behältnisse für Reagentien vorgesehen sein, welche während des Verfahrens zum Analytnachweis mit den Messbereichen in Kontakt gebracht werden.

Auf der Sensorplattform sind vorteilhaft optisch oder mechanisch erkennbare Markierungen zur Erleichterung der Justierung in einem optischen System und / oder zur Verbindung mit der die Ausnehmungen für die Probenbehältnisse enthaltenden Schicht (g) aufgebracht.

Weiterer Gegenstand der Erfindung ist ein analytisches System zur Bestimmung einer oder mehrerer Lumineszenzen entsprechend Anspruch 23.

Dabei ist es von Vorteil, wenn das von der mindestens einen Lichtquelle ausgesandte Anregungslicht kohärent ist und unter dem Resonanzwinkel zur Einkopplung in die optisch transparente Schicht (a) auf die einen oder mehreren Messbereiche eingestrahlt wird.

Um eine Vielzahl von Messbereichen oder alle Messbereiche gleichzeitig anzuregen und deren Lumineszenz gleichzeitig zu bestimmen, wird es bevorzugt, dass das Anregungslicht von mindestens einer Lichtquelle mit einer Aufweitungsoptik zu einem im wesentlichen parallelen Strahlenbündel aufgeweitet wird und unter dem Resonanzwinkel zur Einkopplung in die optisch transparente Schicht (a) auf die einen oder mehreren Messbereiche eingestrahlt wird.

Zur Verminderung von Lumineszenzsignalen ausserhalb der Messbereiche kann es jedoch auch vorteilhaft sein, wenn das Anregungslicht von der mindestens einen Lichtquelle durch ein oder, im Falle mehrerer Lichtquellen, gegebenenfalls mehrere diffraktive optische Elemente, vorzugsweise Dammann-Gitter, oder refraktive optische Elemente, vorzugsweise Mikrolinsen-Arrays, in eine Vielzahl von Einzelstrahlen möglichst gleicher Intensität der von einer gemeinsamen Lichtquelle stammenden Teilstrahlen zerlegt wird, welche jeweils im wesentlichen parallel zueinander auf räumlich getrennte Messbereiche eingestrahlt werden.

Im Falle unzureichender Intensität einer einzigen Lichtquelle oder des Bedarfs nach Lichtquellen unterschiedlicher Emissionswellenlängen, beispielsweise für biologische Applikationen ist es vorteilhaft, wenn als Anregungslichtquellen zwei oder mehrere kohärente Lichtquellen mit gleicher oder unterschiedlicher Emissionswellenlänge verwendet werden.

Um die Signale von einer Vielzahl von Messbereichen getrennt aufzunehmen, wird bevorzugt, dass zur Detektion mindestens ein ortsauflösender Detektor verwendet wird.

Dabei kann als der mindestens eine ortsauflösende Detektor mindestens ein Detektor aus der Gruppe verwendet werden, die von CCD-Kameras, CCD-Chips, Photodioden-Arrays, Avalanche-Dioden-Arrays, Multichannelplates und Vielkanal-Photomultipliern gebildet wird.

In dem erfindungsgemässen analytischen System nach einer der genannten Ausführungen können zwischen der einen oder mehreren Anregungslichtquellen und der Sensorplattform nach einer der vorgenannten Ausführungen und /oder zwischen besagter Sensorplattform und dem einen oder mehreren Detektoren optische Komponenten aus der Gruppe verwendet werden, die von Linsen oder Linsensystemen zur Formgestaltung der übertragenen Lichtbündel, planaren oder gekrümmten Spiegeln zur Umlenkung und gegebenenfalls zusätzlich zur Formgestaltung von Lichtbündeln, Prismen zur Umlenkung und gegebenenfalls zur spektralen Aufteilung von Lichtbündeln, dichroischen Spiegeln zur spektral selektiven Umlenkung von Teilen von Lichtbündeln, Neutralfiltern zur Regelung der übertragenen Lichtintensität, optischen Filtern oder Monochromatoren zur spektral selektiven Übertragung von Teilen von Lichtbündeln oder polarisationsselektiven Elementen zur Auswahl diskreter Polarisationsrichtungen des Anregungs- oder Lumineszenzlichts gebildet werden.

Für eine Reihe von Anwendungen ist es von Vorteil, wenn die Einstrahlung des Anregungslichts in Pulsen mit einer Dauer zwischen 1 fsec und 10 Minuten erfolgt.

Für kinetische Messungen oder für die Diskriminierung rasch abklingender Fluoreszenz von fluoreszenten Verunreinigungen in der Probe oder in Materialien von Komponenten des optischen Systems oder der Sensorplattform selbst kann es von Vorteil sein, wenn das Emissionslicht aus den Messbereichen zeitlich aufgelöst gemessen wird.

Weiterhin wird es bevorzugt, dass das erfindungsgemässe optische System Komponenten umfasst, mit denen zur Referenzierung des verfügbaren Anregungslichts Lichtsignale aus der Gruppe gemessen werden, die von Anregungslicht am Ort der Lichtquellen oder nach ihrer Aufweitung oder nach ihrer Unterteilung in Teilstrahlen, Streulicht bei der Anregungswellenlänge aus dem Bereich der einen oder mehreren räumlich getrennten Messbereiche, und über die Gitterstruktur (c) neben den Messbereichen ausgekoppeltem Licht der Anregungswellenlänge gebildet werden. Besonders von Vorteil ist dabei, wenn die Messbereiche zur Bestimmung des Emissionslichts und des Referenzsignals identisch sind.

Es ist auch möglich, dass die Einstrahlung des Anregungslichts auf und Detektion des Emissionslichts von einem oder mehreren Messbereichen sequentiell für einzelne oder mehrere Messbereiche erfolgt. Dabei kann die sequentielle Anregung und Detektion unter Verwendung beweglicher optischer Komponenten erfolgen, die aus der Gruppe von Spiegeln, Umlenkprismen und dichroischen Spiegeln gebildet wird. Üblicherweise werden für sequentielle Anregung und Detektion in bioanalytischen Array-Systemen kommerziell erhältliche sogenannte Scanner eingesetzt, mit denen, meistens mittels beweglicher Spiegel, ein Anregungslichtstrahl sequentiell über die zu untersuchende Fläche geführt wird. Bei den meisten Scannersystemen ändert sich dabei der Winkel zwischen der beleuchteten Fläche und dem Anregungslichtstrahl. Zur Erfüllung der Resonanzbedingung für die Einkopplung des Anregungslichts in die wellenleitende Schicht der erfindungsgemässen Sensorplattform muss dieser Winkel jedoch im wesentlichen konstant bleiben, d. h. ein in dem erfindungsgemässen optischen System zu verwendender Scanner muss winkelgetreu arbeiten. Diese Forderung wird von einigen kommerziell erhältlichen Scannern erfüllt. Zugleich darf sich jedoch auch nicht die Grösse der angeregten Fläche auf der Sensorplattform ändern. Daher ist ein weiterer Gegenstand dieser Erfindung ein optisches System, welches dadurch gekennzeichnet ist, dass sequentielle Anregung und Detektion unter Verwendung eines im wesentlichen winkel- und fokusgetreuen Scanners erfolgt.

Eine andere mögliche Ausführungsform ist, dass die Sensorplattform zwischen Schritten der sequentiellen Anregung und Detektion bewegt wird. In diesem Fall können die eine oder mehreren Anregungslichtquellen und die zur Detektion verwendeten Komponenten räumlich fixiert sein.

Weiterer Gegenstand der Erfindung ist ein Verfahren zum Lumineszenznachweis eines oder mehrerer Analyten entsprechend Anspruch 31.

Dabei wird bevorzugt, dass die besagten Messbereiche in optischer Wechselwirkung mit dem evaneszenten Feld von in dem planaren optischen Wellenleiter geführtem Anregungslicht stehen.

Bei den vorgenannten Verfahren ist es möglich, dass (1) die isotrop abgestrahlte Lumineszenz oder (2) in die optisch transparente Schicht (a) eingekoppelte und über die Gitterstruktur (c) ausgekoppelte Lumineszenz oder Lumineszenzen beider Anteile (1) und (2) gleichzeitig gemessen werden.

In dem erfindungsgemässen Verfahren kann zur Erzeugung der Lumineszenz oder Fluoreszenz ein Lumineszenz- oder Fluoreszenzlabel verwendet werden, das bei einer Wellenlänge zwischen 300 nm und 1100 nm angeregt werden kann und emittiert.

Bei den Lumineszenz- oder Fluoreszenzlabeln kann es sich um herkömmliche Lumineszenz- oder Fluoreszenzfarbstoffe oder auch um sogenannte lumineszente oder fluoreszente Nanopartikel, basierend auf Halbleitern (W. C. W. Chan und S. Nie, "Quantum dot bioconjugates for ultrasensitive nonisotopic detection", Science 281 (1998) 2016 - 2018) handeln.

Das Lumineszenzlabel kann an den Analyten oder in einem kompetitiven Assay an einen Analogen des Analyten oder in einem mehrstufigen Assay an einen der Bindungspartner der immobilisierten biologischen oder biochemischen oder synthetischen Erkennungselemente oder an die biologischen oder biochemischen oder synthetischen Erkennungselemente gebunden sein.

Zusätzlich können ein zweites oder noch weitere Lumineszenzlabel mit gleicher oder unterschiedlicher Anregungswellenlänge wie das erste Lumineszenzlabel und gleicher oder unterschiedlicher Emissionswellenlänge verwendet werden. Hierbei kann es vorteilhaft sein, wenn das zweite oder noch weitere Lumineszenzlabel bei der gleichen Wellenlänge wie das erste Lumineszenzlabel angeregt werden können, aber bei anderen Wellenlängen emittieren.

Für andere Applikationen ist es vorteilhaft, wenn die Anregungsspektren und Emissionsspektren der eingesetzten Lumineszenzlabel nur wenig oder gar nicht überlappen.

In dem erfmdungsgemässen Verfahren kann es weiterhin vorteilhaft sein, wenn zum Nachweis des Analyten Ladungs- oder optischer Energietransfer von einem als Donor dienenden ersten Lumineszenzlabel zu einem als Akzeptor dienenden zweiten Lumineszenzlabel verwendet wird.

Zusätzlich kann es von Vorteil sein, wenn neben der Bestimmung einer oder mehrerer Lumineszenzen Änderungen des effektiven Brechungsindex auf den Messbereichen bestimmt werden. Von weiterem Vorteil kann dabei sein, wenn die einen oder mehreren Lumineszenzen und / oder Bestimmungen von Lichtsignalen bei der Anregungswellenlänge polarisationsselektiv vorgenommen werden.Weiterhin erlaubt das Verfahren die Möglichkeit, dass die einen oder mehreren Lumineszenzen bei einer anderen Polarisation als der des Anregungslichts gemessen werden.

Das erfindungsgemässe Verfahren nach einer der voranstehenden Ausführungsformen ermöglicht eine gleichzeitige oder sequentielle, quantitative oder qualitative Bestimmung eines oder mehrerer Analyten aus der Gruppe von Antikörpern oder Antigenen, Rezeptoren oder Liganden, Chelatoren oder "Histidin-Tag-Komponenten", Oligonukleotiden, DNA- oder RNA-Strängen, DNA- oder RNA-Analoga, Enzymen, Enymcofaktoren oder Inhibitoren, Lektinen und Kohlehydraten.

Das oben beschriebene Verfahren erlaubt es, direkt biologisches Material ohne biologische oder biochemische Vervielfältigungsmechanismen (z.B. das Polymerase Chain Reaction (PCR)) zu bestimmen. Neben der Vereinfachung der Vorgehensweise wird hier eine lineare Korrelation vom mRNA und beobachtetem Signal erreicht. Das bedeutet, dass auch sog. "rare messages", d.h. biologisch relevante mRNAs, die in sehr geringen Konzentrationen auftreten, hochempfindlich und quantitativ bestimmt werden können. Die parallele Durchführung der Messungen auf einer 96 Well Plate Plattform ermöglicht es, Ergebnisse in bisher unbekannter Qualität und Durchsatz zu erhalten (siehe Ausführungsbeispiel 1e). Als Konsequenz ist daher klar ersichtlich, dass die bei präklinischen und klinischen Studien anfallenden grossen Probemengen sehr viel schneller und präziser als mit den bis anhin verwendeten manuellen Verfahren abgearbeitet werden können. Die Detektion zweier Fluoreszenzmarker auf einem Substanzfleck ermöglicht zudem die direkte Bestimmung der relativen Induktion in einer Einzelmessung (siehe Beispiel 2e). Eine Referenzierung von Chip zu Chip ist deshalb nicht notwendig. Die Qualitätsansprüche an Batch-zu-Batch-Reproduzierbarkeit der Sensorplattformen können deshalb signifikant reduziert werden.

Die zu untersuchenden Proben können natürlich vorkommende Körperflüssigkeiten wie Blut, Serum, Plasma, Lymphe oder Gewebeflüssigkeiten oder Urin oder Eigelb sein.

Eine zu untersuchende Probe kann aber auch eine optisch trübe Flüssigkeit, Oberflächenwasser, ein Boden- oder Pflanzenextrakt, eine Bio- oder Syntheseprozessbrühe sein.

Die zu untersuchenden Proben können auch aus biologischen Gewebeteilen entnommen sein.

Weiterer Gegenstand der vorliegenden Erfindung ist die Verwendung einer Vorrichtung nach einem der Ansprüche 1-22 oder des analytischen Systems nach einem der Ansprüche 23-30.

Mit den nachfolgenden Ausführungsbeispielen soll die Erfindung genauer erläutert und demonstriert werden.

### Beispiel 1: Vorrichtung und Verfahren zur gleichzeitigen Bestimmung von Unterschieden im Expressionsmuster ausgewählter m-RNA's aus verschiedenen Positionen von Tumorgeweben

An verschiedenen Positionen eines extrahierten Tumors - in diesem beispiel einer Prostata Drüse - wird Gewebe entnommen und auf Unterschiede im Expressionsmuster ausgewählter mRNAs als Funktion der Position im Tumorgewebe untersucht. Die Ergebnisse werden eine unabhängig von histologischen Untersuchungen auf reinen genetischen Informationen basierende Aussage erlauben. Die biologische Relevanz eines solchen Verfahrens und Aspekte der Probenvorbereitung sind in der Literatur beschrieben (Kristina A. Cole, David B. Krizmann, and Michael R. Emmert-Buck, "The genetics of cancer - a 3D model" Nature Genetics Supplement 21 (1999) 38-41).

### Herstellung und Aufbringung der biologischen Erkennungselemente auf einer Sensorplattform einer Vorrichtung mit je 400 diskreten Messbereichen in 96 Probenbehältnissen

a) Klone von 100 Genen die - basierend auf in der Literatur beschriebenen Ergebnissen - eine Rolle in der Karzinogenese oder bei Enzündungsreaktionen spielen (siehe Kristina A. Cole, David B. Krizmann, and Michael R. Emmert-Buck, "The genetics of cancer - a 3D model", Nature Genetics Supplement 21 (1999) 38-41, sowie dort zitierte weiterführende Literatur), sowie von solchen Genen, die typischerweise keine Rolle in diesen Prozessen spielen, deren Expresssionsniveau im Idealfall konstant bleibt und deshalb zur Standardisierung herangezogen werden können (sogenannte "Housekeeping Genes" wie beispielsweise β-Actin, GAPDH, Tubulin-a, Ubiquitin, Phospholipase A2, etc.), werden ausgewählt als biologische Erkennungselemente, welche auf der Sensorplattform in diskreten Messbereichen immobilisiert werden sollen.
   Zu der ersten Gruppe ausgewählter potentiell krankheitsrelevanter Gene gehören unter anderem Serin/Threonin-Kinase (STK2), die beta-Untereinheit von Proteasom (PSMB4), CD36-Antigen (Kollagen Typ I Rezeptor, Thrombospondin Rezeptor), Phospholipase A2 (PLA2G1B), Ribosomales Protein L17 (RPL17), Desmoglein 2 (DSG2), Tyro 3 Protein-Tyrosin-Kinase (TYRO3), Typ IV Kollagen (COL4A4), "Activating Transcription Factor 3" (ATF3), Annexin 1 (ANX), weitere Reparaturenzyme, Kinasen etc.
   Das erforderliche genetische Material kann in Plasmidform aus dem UniGene Set NIHs Cancer Genome Anatomy Projects oder alternativ aus kommerziellen Quellen, die das Material des I.M.A.G.E. Konsortiums vertreiben, bezogen werden. Die Überführung und Amplifikation der Gensequenzen im Plasmid zu den entsprechenden cDNAs erfolgt mittels der entsprechenden Primerpaare und erzeugt doppelsträngige Moleküle (Probe cDNAs) mit einer Länge zwischen etwa 300 und 1000 Basenpaaren.
b) Es wird eine Sensorplattform mit den äusseren Abmessungen 76 mm Breite x 112 mm Länge x 0.7 mm Dicke verwendet, auf deren Fläche die 96 Wells mit den Innendimensionen 7 x 7 mm einer klassischen Mikrotiterplatte Platz finden. Das Substratmaterial (optisch transparente Schicht (b)) besteht aus AF 45 Glas (Brechungsindex n = 1.52 bei 633 nm). Im Substrat wird mittels holographischer Belichtung der mit aufgeschleudertem Photolack bedeckten Schicht (b) und anschliessendes nasschemisches Ätzen eine durchgehende Struktur eines Oberflächenreliefgitters einer Periode von 360 nm und einer Tiefe von 25 +/- 5 nm erzeugt, mit Orientierung der Gitterlinien parallel zur ausgewiesenen Breite der Sensorplattform. Die wellenleitende, optisch transparente Schicht (a) aus Ta₂Oₛ auf der optisch transparenten Schicht (b) wird durch reaktives, magnetfeldunterstütztes DC-Sputtern (siehe DE-A-44 10258) erzeugt und hat einen Brechungsindex von 2.15 bei 633 nm (Schichtdicke 150 nm). Infolge des Abscheidungsprozesses überträgt sich die Gitterstruktur auf die Oberfläche der wellenleitenden Schicht nahezu masstäblich im Verhältnis 1:1.
   Zur Vorbereitung auf die Immobilisierung der biochemischen oder biologischen oder synthetischen Erkennungselemente wird die Sensorplattformen mit Chloroform im Ultraschallgerät gereinigt und mittels einer Silanisierungsreaktion mit Glycidyloxypropyltrimethoxysilan chemisch aktiviert und so zur Immobilisierung der Probe cDNAs als biologische Erkennungselemente vorbereitet. Die einzelnen cDNAs in einer Konzentration von 50 ng/µl werden mittels einer kommerziellen piezogesteuerten Mikropipette (GeSim GmbH, Großerkmannsdorf, DE) mit jeweils 10 Tropfen zu 0.1 nl auf einen Punkt aufgebracht, was zu Spotdurchmessern als diskreten Messbereichen von ca. 150 µm führt. In einer 20 x 20 Spot-Anordnung (400 Feature Array) werden jeweils 4 Messbereiche mit gleicher cDNA erzeugt, so dass am Ende des Immobilisierungsvorganges die durch einen statistischen Algorithmus festgelegte Position der 100 verschiedenen als Erkennungselementen dienenden cDNAs bekannt, jedoch rein statistisch vorgegeben ist. Die Spots weisen einen Abstand der Spotmittelpunkte von 300 µm auf und belegen so eine Fläche von 6 x 6 mm auf der Sensorplattform, welche mit der damit in Kontakt stehenden, dicht verschliessenden Schicht (g) aus Polydimethylsiloxan (PDMS) mit entsprechenden, die Arrays umschliessenden quadratischen Ausnehmungen von 7 mm x 7mm eine 96-Well-Platte bildet. Auf der Sensorplattform wird am Boden sämtlicher 96 Probenbehältnisse auf diese Weise ein identisches Raster diskreter Messbereiche erzeugt. Durch thermische Behandlung - Aufheizen auf 60 °C für 30 min. - kann die Immobilisierung optimiert werden.

### c) Probenmaterial:

An verschiedenen Positionen einer Prostatadrüse (sowohl im phänotypisch auffallenden wie auch im phänotypisch nicht auffallenden Gewebe) wird Gewebematerial (ca 50 000 bis 100000 Zellen) z. B. in Form von Gewebefeinschnitten entnommen. Das Gewebe wird in gefrorenem Zustand mechanisch aufgeschlossen und über eine Standard Phenol / Chloroform-Extraktion wird "Total RNA" isoliert. Mittels einer Oligotex Spin Column (Qiagen, Hilden, DE) wird mRNA aus der Total RNA Fraktion isoliert. Mittels eines Reversed Transkriptase Verfahrens (z.B. Life Technologies) unter Anwesenheit einer Cy5 (Pharmacia Amersham) markierten Nukleobase (Cy5-dUTP) werden sämtliche mRNA Arten in fluoreszenzmarkierte cDNA Moleküle übergeführt. Das so erhaltene Material wird in Hybridisierungspuffer aufgenommen, so daß eine Gesamtkonzentration von maximal 10 ng/µl vorliegt als flüssige Probe.

### d) Analytisches System

### Optisches System

### i) Anregungseinheit

Die Sensorplattform ist auf einer computergesteuerten Justiereinheit montiert, welche die Translation parallel und senkrecht zu den Gitterlinien sowie eine Rotation Drehachse parallel zu den Gitterlinien und durch den Schwerpunkt des auf die Gitterstruktur (I) auftreffenden Anregungslichtflecks zur Einkopplung in die in Beispiel 1b genannte Sensorplattform erlaubt. Unmittelbar nach dem als Anregungslichtquelle benutzten Laser befindet sich im Lichtweg ein Shutter, um den Lichtweg zu blockieren, wenn keine Messdaten aufgenommen werden sollen. Zusätzlich können Neutralfilter oder Polarisatoren an dieser Stelle oder auch anderen Positionen im weiteren Weg des Anregungslichts zur Sensorplattform in den Lichtweg gestellt werden, um die Anregungsintensität stufenweise oder kontinuierlich zu variieren.

Der Anregungslichtstrahl eines Helium-Neon-Lasers bei 632.8 nm wird mit einer 25-fachen Aufweitungsoptik auf ein paralleles Strahlenbündel mit kreisförmigem Querschnitt von 25 mm Durchmesser aufgeweitet. Hiervon wird eine quadratische Fläche von 10 mm Länge x 10 mm Breite (entsprechend den Bezeichnungen für die Gitterstrukturen) aus dem zentralen Teil des Anregungslichtbündels ausgewählt und über einen dichroischen Spiegel (650 DRLP, Omega Optical, Brattleborough, VT, USA) durch die optisch transparente Schicht (a) hindurch auf eines der 96 Array im Zentrum des Anregungslichtbündels geleitet. Die Sensorplattform wird auf maximale Einkopplung justiert, welche an einem der Minimum der Transmission von Anregungslicht durch die Sensorplattform und einem Maximum der Summe aus reflektiertem Anregungslicht und innerhalb der Messgenauigkeiten unter gleichem Winkel wie dem der Reflexion nach Einkopplung in die Sensorplattform über die durchgehende Gitterstruktur (c) wieder ausgekoppeltem Anregungslicht erkennbar ist. Der optimale Einkopplungswinkel kann sowohl durch visuelle Kontrolle als auch computergesteuert über die Signale von in den entsprechenden Strahlengängen positionierten Photodioden mit anschliessendem Verstärker bestimmt werden. Auf diese Weise wird als Resonanzwinkel für die Einkopplung ein Winkel von +3.5° bestimmt.

### ii) Detektion

Als ortsauflösender Detektor dient eine CCD-Kamera (TE3/A Astrocam, Cambridge, UK) mit Peltier-Kühlung (Betriebstemperatur -30 °C). Die Signalerfassung und Fokussierung auf den CCD-Chip erfolgt mit einem Heligon-Tandem-Objektiv (Rodenstock, zweimal Cine-Heligon 2.1/100 mm). Zwischen den beiden Hälften des Heligon-Tandem-Objektivs befinden sich, auf einem Filterwechsler montiert, 2 Interferenzfilter (Omega, Brattleborough, Vermont, USA) mit Zentralwellenlänge von 680 nm und 40 nm Bandbreite, sowie entweder ein Neutralfilter (zur Transmission des abgeschwächten, gestreuten Anregungs- und sehr viel schwächeren Lumineszenzlichts von den Messbereichen) oder ein Neutralfilter in Kombination mit einem Interferenzfilter (zur Transmission des abgeschwächten , von den Messbereichen gestreuten Anregungslichts) . Die Signale bei der Anregungs- und der Lumineszenzwellenlänge werden alternierend gemessen. Die Auswertung der Daten erfolgt durch kommerziell erhältliche Bildverarbeitungssoftware (ImagePro Plus)

### e) Verfahren zum Lumineszenznachweis der Hybridisierungsreaktion:

In jedes der 96 Probenbehältnisse der oben beschriebenen Vorrichtung mit immobilisierten cDNA Arrays werden 30 µl der wie unter dem Abschnitt "Probematerial" vorbereiteten flüssigen Proben manuell oder über einen Laborroboter (z.B. Beckman 2000) eingefüllt. Die 96 Well Platte wird anschliessend mit einem Deckel hermetisch abgeschlossen, so daß über jedem Well ein Dampfraum von maximal 100 µl entsteht. In einem ersten thermischen Schritt (Temperaturerhöhung auf 85 °C für 30 Minuten) werden die auf der Oberfläche immobilisierten doppelsträngigen cDNAs dehybridisiert (vereinzelt) und so zur Hybridisierung mit den in der Probe vorhandenen Cy5 markierten einzelsträngigen cDNAs vorbereitet. Durch langsames Abkühlen auf 43 °C (etwa 2 °C pro Minute) und 8 Stunden Temperieren auf 43 °C wird eine Hybridisierung von immobilisierten einzelsträngigen cDNA mit den komplementären Cy5 markierten Molekülen unter sog. stringenten Bedingungen (Hybridisierung nur völlig komplementärer Einzelstränge) ermöglicht. Durch Austausch der verbliebenen Probeflüssigkeit und Absenken auf Raumtemperatur kann dieser Zustand stabilisiert werden.

Zum Auslesen der Arrays in den einzelnen Probenbehältnissen wird die vorgehend beschriebene Vorrichtung in dem beschriebenen analytischen System eingesetzt und auf maximale Einkopplung des Anregungslichts im Array des ersten der 96 Probenbehältnisse entsprechend der obigen - Beschreibung justiert, was mit Position des Filterwechslers für die Anregungswellenlänge kontrolliert wird. Anschliessend wird die Intensität des Fluoreszenzlichts aus den Messbereichen (Spots) des Arrays mit Position des Filterwechslers für die Lumineszenzwellenlänge gemessen. Das Auslesen der Arrays in den weiteren Probenbehältnissen erfolgt sequentiell (für jeweils ein Probenbehältnis mit einem Array von 400 diskreten Messbereichen nach dem anderen), mittels Translation der Vorrichtung zur nächsten Position für das Auslesen der Lumineszenzsignale aus dem nächsten Probenbehältnis.

### Beispiel 2: Bestimmung der Art und des Ausmasses von Enzyminduktion in Toxikologie und Drug Metabolismus mittels eines schnellen und hochparallelen auf Mikroarrays basierenden Differenzverfahrens

In der pharmazeutischen Produktentwicklung werden nach Applikation von Xenobiotika (biologisch und / oder pharmakologisch wirksamen chemischen Substanzen) in Versuchstieren und im Menschen Untersuchungen des Wirkstoffmetabolismus und Toxikologiestudien durchgeführt. Dabei werden u. a. im "First Path Metabolismus" die ersten metabolischen Abbaureaktionen in der Leber untersucht. Häufig wird nach der Verabreichung solcher Substanzen eine "Enzyminduktion", d. h. eine Beeinflussung der Konzentration der im Metabolismus in der Leber beteiligten Enzyme, typischerweise aus der Gruppe der P450 Isoenzyme (gemischt funktionelle Monooxygenasen), beobachtet. Diese Änderung wiederum beeinflusst die Qualität und die Geschwindigkeit der weiteren Metabolisierung solcher Substanzen. Es besteht die Notwendigkeit, beim Test neuer pharmakologisch aktiver Substanzen diese Beeinflussung sehr früh im Testverfahren auf der Ebene der mRNA quantitativ und mit hoher Empfindlichkeit im Vergleich zum Metabolismus von unbehandelten (nicht induzierten) Organismen zu messen.

### Herstellung und Aufbringung der biologischen Erkennungselemente auf einer Sensorplattform einer Vorrichtung mit je 100 diskreten Messbereichen in 96 Probenbehältnissen

a) Klone der 20 wichtigsten in der Literatur beschriebenen Cytochrom P450 Isoenzyme, die eine Rolle im "First Path Metabolismus" in der Leber eine Rolle spielen, sowie 5 Klone
   von Genen, die typischerweise keine Rolle in diesen Prozessen spielen, deren Expresssionsniveau im Idealfall konstant bleibt und deshalb zur Standisierung herangezogen werden können (sogenannte "Housekeeping Genes", u.a. β-Actin und GAPDH, Tubulin-a, Ubiquitin, Phospholipase A2, etc.), werden ausgewählt als biologische Erkennungselemente, welche auf der Sensorplattform in diskreten Messbereichen immobilisiert werden sollen.
   Zu der ersten Gruppe augewählter biologischer Markersubstanzen gehören unter anderem CYP1A1, CYP1A2, CYP2B1, CYP2B2, CYP3A1, CYP3A2, CYP4A1.
   Das erforderliche genetische Material kann in Plasmidform kommerziell bezogen werden. Die Überführung und Amplifikation der Gensequenzen im Plasmid zu den entsprechenden cDNAs erfolgt mittels der entsprechenden Primerpaare und erzeugt doppelsträngige Moleküle (Probe cDNAs) mit einer Länge zwischen etwa 300 und 1000 Basenpaaren.
b) Es wird eine Sensorplattform mit den äusseren Abmessungen 76 mm Breite x 112 mm Länge x 0.7 mm Dicke verwendet, auf deren Fläche durch Kombination mit einer Platte aus Polymethylmethacrylat mit in Richtung der Sensorplattform offenen Ausnehmungen
   mit den Innendimensionen 7 mm Breite x 7 mm Länge x 0.15 mm Höhe 96 Mikroflusszellen im Raster einer klassischen Mikrotiterplatte erzeugt werden können. Mittels Dichtungsringen, welche die Ausnehmungen umschliessen, sind die Ausnehmungen gegeneinander dicht verschliessend abgedichtet.

Das Substratmaterial (optisch transparente Schicht (b) besteht aus AF 45 Glas (Brechungsindex n = 1.52 bei 633 nm). Im Substrat wird mittels holographischer Belichtung der mit aufgeschleudertem Photolack bedeckten Schicht (b) und anschliessendes nasschemisches Ätzen ein Raster von Paaren aus Ein- und Auskoppelgittern mit parallel zur Breite der Sensorplattform verlaufenden Gitterlinien (360 nm Periode) von 12 +/- 3 nm Gittertiefe erzeugt, wobei die Gitterlinien über die gesamte Breite der Sensorplattform ausgeprägt sind. Der Abstand zwischen aufeinanderfolgenden Gitterpaaren beträgt 9 mm, die Länge der einzelnen Gitterstrukturen (parallel zur Länge der Sensorplattform) 0.5 mm Der Abstand zwischen dem Ein- und Auskoppelgitter eines Gitterpaares beträgt 5.5 mm, so dass die Ein- und Auskopplung des Anregungslichts jeweils innerhalb des Bereichs der Probenbehältnisse, nach Kombination der Sensorplattform mit der oben beschriebenen Polymethylmethacrylatplatte, erfolgen kann.
Die wellenleitende, optisch transparente Schicht (a) aus Ta₂O₅ auf der optisch transparenten Schicht (b) wird durch reaktives, magnetfeldunterstütztes DC-Sputtern (siehe DE-A- 44 10258) erzeugt und hat einen Brechungsindex von 2.15 bei 633 nm (Schichtdicke 150 nm). Die Gitterstruktur überträgt sich infolge des Abscheidungsprozesses nahezu masstäblich im Verhältnis 1:1 auf die Oberfläche der wellenleitenden Schicht, in die sich die Gitterstruktur überträgt.

Die von der Sensorplattform und der damit kombinierten Polymethylmethacrylplatte gebildeten Probenbehältnisse weisen auf den der Sensorplattform gegenüberliegenden Begrenzungsflächen konisch ausgebohrte Öffnungen auf, so dass eine Befüllung oder Entleerung der Probenbehältnisse durch Einpressen von standardisierten, kommerziell erhältlichen Pipettenspitzen aus Polypropylen erfolgen kann.

Zur Vorbereitung auf die Immobilisierung der biochemischen oder biologischen oder synthetischen Erkennungselemente wird die Sensorplattformen mit Chloroform im Ultraschallgerät gereinigt und mittels einer Silanisierungsreaktion mit Glycidyloxypropyltrimethoxysilan chemisch aktiviert und so zur Immobilisierung der Probe cDNA's als biologische Erkennungselemente vorbereitet. Die einzelnen cDNAs in einer Konzentration von 50 ng/µl werden mittels einer kommerziellen piezogesteuerten Mikropipette (GeSim GmbH, Großerkmannsdorf, DE) mit jeweils 10 Tropfen zu 0.1 nl auf einen Punkt aufgebracht, was zu Spotdurchmessem als diskreten Messbereichen von ca. 150 µm führt. In einer 10 x 10 Spot-Anordnung (100 Feature Array werden jeweils 4 Messbereiche mit gleicher cDNA erzeugt, so dass am Ende des Immobilisierungsvorganges die durch einen statistischen Algorithmus festgelegte Position der 25 verschiedenen als Erkennungselementen dienenden cDNAs bekannt, jedoch rein statistisch vorgegeben ist. Die Spots weisen einen Abstand der Spotmittelpunkte von 400 µm auf und belegen so eine Fläche von 4 x 4 mm auf der Sensorplattform. Durch thermische Behandlung - Aufheizen auf 60 °C für 30 min. - kann die Immobilisierung optimiert werden.

Auf die so vorbereitete Sensorplattform wird die beschriebene Polymethylmethacrylatplatte so aufgebracht, dass die einzelnen Probenbehältnisse gegeneinander über die umgebenden Dichtungen fluidisch dicht verschliessend gegeneinander abgeschlossen und die Arrays aus jeweils 100 von den Spots gebildeten Messbereichen zentrisch am Boden der Probenbehältnisse angeordnet sind.

### c) Probenmaterial:

### i) mRNA aus nicht induzierten (nicht mit Xenobiotika behandelten) Versuchstieren

Etwa 250 mg Leber aus getöteten Mäusen wird mit flüssigem Stickstoff schockgefroren, im Mörser zerkleinert und mit Chloroform entfettet. Anschließend wird "Total RNA" über eine Standard Phenol / Chloroform-Extraktion isoliert. Mittels einer Oligotex Spin Column (Qiagen) wird mRNA aus der Total RNA Fraktion isoliert. Mittels eines Reversed Transkriptase Verfahrens (z.B. Life Technologies) unter Anwesenheit einer Cy5 (Pharmacia Amersham) markierten Nukleobase (Cy5-dUTP) werden sämtliche mRNA-Arten in fluoreszenzmarkierte cDNA Moleküle übergeführt. Das so erhaltene Material wird in Hybridisierungspuffer aufgenommen, so daß eine Gesamtkonzentration von maximal 10 ng/µl als flüssige Probe vorliegt.

### ii) mRNA aus induzierten (mit Xenobiotika behandelten - als Testinduktor eignet sich zum Beispiel Phenobarbital) Versuchstieren

Die Ausführung erfolgt in analoger Weise wie vorgehend unter Beispiel 2.c.i) beschrieben, mit dem einzigen Unterschied, dass die Fluoreszenzmarkierung mit Cy3 (Pharmacia Amersham) anstelle von Cy5 erfolgt.

### iii) Markierte mRNA aus den Ansätzen i) und ii) wird im Verhältnis 1:1 gemischt.

### d) Analytisches System

### Optisches System

### i) Anregungseinheit

Die Sensorplattform ist auf einer computergesteuerten Justiereinheit montiert, welche die Translation parallel und senkrecht zu den Gitterlinien sowie eine Rotation Drehachse parallel zu den Gitterlinien und durch den Schwerpunkt des auf die Gitterstruktur (I) auftreffenden Anregungslichtflecks zur Einkopplung in die in Beispiel 2b) genannte Sensorplattform erlaubt. Unmittelbar nach dem als Anregungslichtquelle benutzten Laser befindet sich im Lichtweg ein Shutter, um den Lichtweg zu blockieren, wenn keine Messdaten aufgenommen werden sollen. Zusätzlich können Neutralfilter oder Polarisatoren an dieser Stelle oder auch anderen Positionen im weiteren Weg des Anregungslichts zur Sensorplattform in den Lichtweg gestellt werden, um die Anregungsintensität stufenweise oder kontinuierlich zu variieren.

Der Anregungslichtstrahl eines Helium-Neon-Lasers (2 mW) wird mit einer Linsenkombination, unter Verwendung einer Zylinderlinse, zu einem spaltförmigem Lichtstrahl (parallel zu den Gitterlinien der Sensorplattform) aufgeweitet. Die oberen und unteren Randgebiete des parallel zu den Gitterlinien leicht divergenten, in der dazu senkrechten Projektion jedoch parallelen Anregungslichts werden durch einen Spalt ausgeblendet. Das resultierende Lichtbündel mit spaltförmigem Querschnitt auf der Gitterstuktur wird auf die rechte Kante des Einkoppelgitters in einem der Probenbehältnisse geleitet. Der Anregungslichtfleck hat eine Grösse von ca. 0.8 mm Länge x 4 mm Breite. Die Sensorplattform wird auf maximale Einkopplung justiert, was an maximaler Intensität des Streulichts erkennbar ist, welches längs des eingekoppelten Modes geführten Anregungslichts durch Streuung abgestrahlt wird. Dieses Maximum kann man sowohl durch visuelle Bestimmung als auch durch Abbildung des mit dem Abbildungssystem erfassten Streulichts längs des Anregungsmodes auf einen optoelektronischen Detektor, z. B. auf die Pixels einer CCD-Kamera als Beispiel eines ortsauflösenden Detektors oder einer Photodiode als Beispiel eines nicht ortsaulösenden Detektors, bestimmen. Unter denselben Einkoppelbedingungen misst man für das am Auskoppelgitter ausgekoppelte im Wellenleiter transmittierte Anregungslicht ebenfalls ein Maximalsignal. Als Resonanzwinkel für die Einkopplung wird ein Winkel von +3.8° bestimmt.

Als zweite Anregungslichtquelle wird ein grüner Helium-Neon-Laser mit Emission bei 543 nm verwendet. Über einen Spiegel, der sowohl manuell als auch motorgetrieben zwischen festen Positionen klappbar ist, kann der Anregungsstrahl in denselben Strahlengang wie der vorgehend beschriebene rote Helium-Neon-Laser-Strahl (633 nm) geleitet werden, wobei dann zugleich der Strahlengangdes roten Lasers blockiert wird.

Die Einstellung des Resonanzwinkels für die Einkopplung folgt in gleicher Weise wie oben beschrieben, wobei ein Wert von +15° bestimmt wird.

### ii) Detektion

Als ortsauflösender Detektor dient eine CCD-Kamera (TE3/A Astrocam, Cambridge, UK) mit Peltier-Kühlung (Betriebstemperatur -30 °C). Die Signalerfassung und Fokussierung auf den CCD-Chip erfolgt mit einem Heligon-Tandem-Objektiv (Rodenstock, zweimal Cine-Heligon 2.1/100 mm). Zwischen den beiden Hälften des Heligon-Tandem-Objektivs befinden sich, auf einem Filterwechsler montiert, 2 Interferenzfilter (Omega, Brattleborough, Vermont) mit Zentralwellenlänge von 680 nm und 40 nm Bandbreite, sowie entweder ein Neutralfilter (zur Transmission des abgeschwächten, gestreuten Anregungs- und sehr viel schwächeren Lumineszenzlichts von den Messbereichen) oder ein Neutralfilter in Kombination mit einem Interferenzfilter (zur Transmission des abgeschwächten , von den Messbereichen gestreuten Anregungslichts). Die Signale bei der Anregungs- und der Lumineszenzwellenlänge werden alternierend gemessen. Die Auswertung der Daten erfolgt durch kommerziell erhältliche Bildverarbeitungssoftware (ImagePro Plus)

### e) Verfahren zum Lumineszenznachweis der Hybridisierungsreaktion:

Jede der 96 Mikroflusszellen wird individuell mit Hilfe des Laborroboters mit nach dem oben aufgeführten Verfahren behandeltem biologischen Material gefüllt. In einem ersten thermischen Schritt (Temperaturerhöhung auf 85 °C für 30 Minuten) werden die auf der Oberfläche immobilisierten doppelsträngigen cDNAs dehybridisiert (vereinzelt) und so zur Hybridisierung mit den in der Probe vorhandenen Cy5 respektive Cy3 markierten einzelsträngigen cDNAs vorbereitet. Durch langsames Abkühlen auf 43 °C (etwa 2 °C pro Minute) und 8 Stunden Temperieren auf 43 °C wird eine Hybridisierung von immobilisierten einzelsträngigen cDNA mit den komplementären Cy5 bzw Cy3 markierten Molekülen unter sogenannten stringenten Bedingungen (Hybridisierung nur völlig komplementärer Einzelstränge), jedoch ohne kinetische oder thermodynamische Bevorzugung der verschieden markierten Moleküle, ermöglicht. In einem weiteren Schritt wird mit Hilfe des Laborroboters die überstehende Flüssigkeit durch reinen Hybridisierungspuffer ersetzt, um nicht gebundenes, zur Fluoreszenz anregbares Material sowie unspezifisch an der Oberfläche gebundenes Material zu entfernen. Nach der Lumineszenzmessung kann zur Regenerierung mit Hilfe des Laborroboters 2-molare Harnstofflösung als chaotropes Reagenz zugeführt werden. Eine Erhöhung der Temperatur auf bis zu 80 °C vervollständigt diesen Prozess. Nach weiterem Spülen mit 10 mM Phosphatpuffer und Reäquilibrieren mit reinem Hybridisierungspuffer kann das Fluidik / Arraysystem wiederbenutzt werden.

Zum Auslesen der Arrays in den einzelnen Probenbehältnissen wird die vorgehend beschriebene Vorrichtung in dem beschriebenen analytischen System eingesetzt und auf maximale Einkopplung des Anregungslichts (zunächst für 633 nm) im Array des ersten der 96 Probenbehältnisse entsprechend der obigen Beschreibung justiert, was mit Position des Filterwechslers für die Anregungswellenlänge kontrolliert wird. Anschliessend wird die Intensität des Fluoreszenzlichts aus den Messbereichen (Spots) des Arrays mit Position des Filterwechslers für die Lumineszenzwellenlänge (zunächst mit Filter 680 DF 40, Omega Brattleborough, VT, für Cy5) gemessen. Das Auslesen der Arrays in den weiteren Probenbehältnissen erfolgt sequentiell (für jeweils ein Probenbehältnis mit einem Array von 100 diskreten Messbereichen nach dem anderen), mittels Translation der Vorrichtung zur nächsten Position für das Auslesen der Lumineszenzsignale aus dem nächsten Probenbehältnis.

Anschliessend wird die Vorrichtung auf die Einkoppleposition für das Array im ersten Probenbehältnis zurückgefahren und nach Umklappen des Spiegels auf maximale Einkopplung für das grüne Anregungslicht (543 nm) eingestellt. Anschliessend wird die Intensität des Fluoreszenzlichts aus den Messbereichen (Spots) des Arrays mit Position des Filterwechslers für die kürzerwellige Lumineszenzwellenlänge (mit Filter 580 DF 40, Omega Brattleborough, VT, USA, für Cy3) gemessen. Das Auslesen der Arrays in den weiteren Probenbehältnissen erfolgt sequentiell (für jeweils ein Probenbehältnis mit einem Array von 100 diskreten Messbereichen nach dem anderen), mittels Translation der Vorrichtung zur nächsten Position für das Auslesen der Lumineszenzsignale aus dem nächsten Probenbehältnis.

### Beispiel 3: Vorrichtung und Verfahren zur gleichzeitigen quantitativen Bestimmung mehrerer Zytokin-Markerproteine in einer und mehrerer Analysenproben (Multi-Zytokin Platte)

a) Als Vorrichtung dient eine quadratische Sensorplattform mit den äusseren Abmessungen 101.6 mm x 101.6 mm (4 Zoll x 4 Zoll) x 0.7 mm Dicke, verbunden mit einer 5 mm starken PDMS-Schicht (Polydimethylsilikon, Sylgard 184 Silicon Elastomer Kit von Dow Corning), in der offene, runde Ausparungen von jeweils 7 mm Durchmesser, in einem (Zentrum-zu-Zentrum) Abstand von 9 mm, für die Probenbehältnisse zur Aufnahme von Analysenvolumina (10-100 µl) geformt wurden. Die Ausparungen sind in Form von 11 Reihen und 11 Spalten in der Schicht organisiert (total 121 Wells). Zur Unterdrückung von Streulicht wurde das Polymermaterial mit schwarzem Pigment eingefärbt.
   Das Substratmaterial der Sensorplattform (optisch transparente Schicht (b) besteht aus AF 45 Glas (Brechungsindex n = 1.52 bei 633 nm). Im Substrat wird mittels holographischer Belichtung der mit aufgeschleudertem Photolack bedeckten Schicht (b) und anschliessendes nasschemisches Ätzen eine durchgehende Struktur eines Oberflächenreliefgitters einer Periode von 360 nm und einer Tiefe von 25 +/- 5 nm erzeugt, mit Orientierung der Gitterlinien parallel zur Kante der Sensorplattform. Die wellenleitende, optisch transparente Schicht (a) aus Ta₂O₅ auf der optisch transparenten Schicht (b) wird durch reaktives, magnetfeldunterstütztes DC-Sputtern (siehe DE 4410258) erzeugt und hat einen Brechungsindex von 2.15 bei 633 nm (Schichtdicke 150 nm). Die Gittertiefen auf der wellenleitenden Schicht, in die sich die Gitterstruktur infolge des Abscheidungsprozesses nahezu masstäblich im Verhältnis 1:1 1 überträgt.
   Die Oberfläche der Sensorplattform wird unter Einwirkung eines Sauerstoffplasmas gereinigt, um organische Kontamination zu entfernen. Anschliessend wird eine Haftvermittlungsschicht, bestehend aus einem Monolayer von Mono-Octadecylphosphat, durch einen Selbstorganisierungsprozess (Self-Assembly) auf die hydrophile Wellenleiteroberfläche aufgebracht. Diese Oberflächenmodifizierung führt zu einer sehr hydrophoben Oberfläche (Kontaktwinkel 100° -110° gegenüber Wasser). Der Prozess der Oberflächenmodifizierung wurde in der Literatur näher beschrieben (D. Brovelli et al., Langmuir 15 (1999) 4324 - 4327).
   Auf die hydrophobe Oberfläche der mit der Haftvermittlungsschicht versehenen Sensorplattform werden 121 Arrays (in 11 Reihen x 11 Spalten) von je 25 Spots, ihrerseits in einer Anordnung von jeweils 5 Reihen x 5 Spalten, mit unterschiedlichen Antikörpern als biologischen Erkennungselementen zur Erkennung und Bindung von Zytokin-Markerproteinen mit einem Inkjet Plotter, Modell NP1C (Firma GeSiM mbH, Grosserkmannsdorf, DE) aufgebracht.
b) Die Antikörper werden in einer Konzentration von 100 µg/ml in phosphatgepufferter Salzlösung (pH 7.4) mit einem Anteil von Zucker (0.1 % Trehalose) aufgetragen, in flüssigem Zustand für 2-3 Stunden in gesättigter Wasserdampfatmosphäre inkubiert, anschliessend gewaschen und mit Stickstoff trocken geblasen. Der Durchmesser der Spots, mit einem Abstand (Zentrum-zu-Zentrum) von 1 mm, beträgt im Mittel 0.5 mm. Fünf verschiedene Antikörper zur Erkennung von Zytokinen, im speziellen von unterschiedlichen Interleukinen, werden in den jeweils 5 Reihen eines Einzelarrays verwendet. Um aus jeder Einzelmessung pro zuzuführender Probe zugleich bereits Daten zur statistischen Assayreproduzierbarkeit zu erhalten, werden pro Array jeweils 5 Messbereiche mit gleichen Interleukin-Antikörpern als biologische Erkennungselemente erzeugt. Dabei wird die Position des Messbereichs mit dem jeweiligen Interleukin-Antikörper als biologischem Erkennungselement durch einen statistischen Algorhythmus festgelegt, so dass am Ende des Immobiliserungsvorganges die Positionen der 5 verschiedenen Interleukine im 25-Spot Array bekannt, jedoch rein statistisch vorgegeben sind. Damit kann in dem nachfolgenden Nachweisverfahren eine bedeutende Einsparung an Analysenzeit und einzusetzender Probenmenge im Vergleich zu den beschriebenen bekannten Nachweisverfahren (z. B. ELISA) erzielt werden, welche zur Bestimmung der Assayreproduzierbarkeit im allgemeinen eine Vielzahl von Einzelmessungen in diskreten Probenbehältnissen und aufgrund der notwendigen grossen Anzahl von Einzelmessungen auch auf mehreren Mikrotiterplatten voraussetzen.
   Die verbleibende, nicht protein-bedeckte hydrophobe Oberfläche wird mit 10 mg/ml Serumalbumin (Inkubationszeit 10 min), zur Unterdrückung von unspezifischer Bindung von Sekundärantikörpern im späteren Nachweisverfahren, geblockt. Nach erfolgter Antikörperauftragung wird die PDMS-Schicht mit der präparierten Sensorplattform derart verbunden, dass sich jeweils ein Array innerhalb der 121 Aussparungen der PDMS-Schicht befindet.

### c) Analytisches System

### Optisches System

### i) Anregungseinheit

Die Sensorplattform ist auf einer computergesteuerten Justiereinheit montiert, welche die Translation parallel und senkrecht zu den Gitterlinien sowie eine Rotation Drehachse parallel zu den Gitterlinien und durch den Schwerpunkt des auf die Gitterstruktur (I) auftreffenden Anregungslichtflecks zur Einkopplung in die in Beispiel 3a) genannte Sensorplattform erlaubt. Unmittelbar nach dem als Anregungslichtquelle benutzten Laser befindet sich im Lichtweg ein Shutter, um den Lichtweg zu blockieren, wenn keine Messdaten aufgenommen werden sollen. Zusätzlich können Neutralfilter oder Polarisatoren an dieser Stelle oder auch anderen Positionen im weiteren Weg des Anregungslichts zur Sensorplattform in den Lichtweg gestellt werden, um die Anregungsintensität stufenweise oder kontinuierlich zu variieren.

Der Anregungslichtstrahl eines Helium-Neon-Lasers bei 632.8 nm wird mit einer 25-fachen Aufweitungsoptik auf ein paralleles Strahlenbündel mit kreisförmigem Querschnitt von 25 mm Durchmesser aufgeweitet. Hiervon wird eine quadratische Fläche von 16 mm Länge x 16 mm Breite (entsprechend den Bezeichnungen für die Gitterstrukturen) aus dem zentralen Teil des Anregungslichtbündels ausgewählt und über einen dichroischen Spiegel (650 DRLP, Omega Optical, Brattleborough, VT, USA) durch die optisch transparente Schicht (a) hindurch auf eine Gruppe von 4 der 121 Arrays im Zentrum des Anregungslichtbündels geleitet. Die Sensorplattform wird auf maximale Einkopplung justiert, welche an einem der Minimum der Transmission von Anregungslicht durch die Sensorplattform und einem Maximum der Summe aus reflektiertem Anregungslicht und innerhalb der Messgenauigkeiten unter gleichem Winkel wie dem der Reflexion nach Einkopplung in die Sensorplattform über die durchgehende Gitterstruktur (c) wieder ausgekoppeltem Anregungslicht erkennbar ist. Der optimale Einkopplungswinkel kann sowohl durch visuelle Kontrolle als auch computergesteuert über die Signale von in den entsprechenden Strahlengängen positionierten Photodioden mit anschliessendem Verstärker bestimmt werden. Auf diese Weise wird als Resonanzwinkel für die Einkopplung ein Winkel von +3.5° bestimmt.

### ü) Detektion

Als ortsauflösender Detektor dient eine CCD-Kamera (TE3/A Astrocam, Cambridge, UK) mit Peltier-Kühlung (Betriebstemperatur -30 °C). Die Signalerfassung und Fokussierung auf den CCD-Chip erfolgt mit einem Heligon-Tandem-Objektiv (Rodenstock, zweimal Cine-Heligon 2.1/100 mm). Zwischen den beiden Hälften des Heligon-Tandem-Objektivs befinden sich, auf einem Filterwechsler montiert, 2 Interferenzfilter (Omega, Brattleborough, Vermont) mit Zentralwellenlänge von 680 nm und 40 nm Bandbreite, sowie entweder ein Neutralfilter (zur Transmission des abgeschwächten, gestreuten Anregungs- und sehr viel schwächeren Lumineszenzlichts von den Messbereichen) oder ein Neutralfilter in Kombination mit einem Interferenzfilter (zur Transmission des abgeschwächten , von den Messbereichen gestreuten Anregungslichts) . Die Signale bei der Anregungs- und der Lumineszenzwellenlänge werden alternierend gemessen. Die Auswertung der Daten erfolgt durch kommerziell erhältliche Bildverarbeitungssoftware (ImagePro Plus)

### d) Nachweisverfahren

Zur spezifischen Erkennung der nachzuweisenden Zytokine wird das Format eines Sandwich-Assays gewählt. Für die gewählten Zytokine (Interleukine IL#1 bis 11#5) werden jeweils 11 Kalibrationslösungen in phosphatgepufferter Salzlösung mit einem Zusatz von 0.1% Serumalbumin und 0.05% Tween20 hergestellt (Interleukinmengen 0, 0.5, 1, 2, 5, 10, 20, 50, 100, 200, 300 µg/ml). Zusätzlich werden 11 Misch-Kalibrationslösungen aller 5 Interleukine hergestellt, welche jedes der 5 Interleukine in gleicher Konzentration enthalten, sowie über ein Zufallsverfahren 55 Probelösungen, welche unbekannte Konzentrationen der Interleukine enthalten. Dann werden die individuellen Konzentrationslösungen mit dem entsprechenden spezifischen biotinylierten sekundären Interleukin-Antikörper (jeweils 200-picomolar) eine Stunde lang vorinkubiert. Entsprechend werden die Misch-Kalibrationslösungen sowie die Probelösungen mit einer Mischung der 5 verschiedenen sekundären biotinylierten Anti-Interleukin-Antikörper (jeweils 200-picomolar) ebenfalls eine Stunde lang vorinkubiert.

Dann werden je 50 µl der individuellen Kalibrationslösungen in die ersten 5 Reihen von Probenbehältnissen auf der Sensorplattform gefüllt, so dass im nachfolgenden Fluoreszenznachweisschritt mit jeder Reihe eine individuelle Kalibrationskurve (Dose-Response Curve) pro Interleukin erzeugt werden kann. In die sechste Reihe von 11 Probenbehältnissen werden je 50 µl der Misch-Kalibrationslösungen gefüllt, um später entsprechende Kalibrationskurven in Anwesenheit der Analytgemische zu erzeugen. Die 55 Probelösungen werden entsprechend auf die übrigen 5 Reihen von Probenbehältnissen verteilt. Die Inkubationszeit mit allen 121 Messlösungen beträgt 30 Minuten. Nachfolgend werden die Probenbehältnisse mit Puffer (phosphatgepufferte Salzlösung mit einem Zusatz von 0.1% Serumalbumin und 0.05% Tween20) gewaschen. Im abschliessenden Fluoreszenznachweisschritt werden in jedes Probenbehältnis 50 µl von 1-nanomolarem Cy5-Streptavidin (Amersham-Pharmacia) in Pufferlösung (phosphatgepufferter Salzlösung mit einem Zusatz von 0.1% Serumalbumin und 0.05% Tween20) gefüllt und nach weiterer 15-minütiger Inkubationszeit die Fluoreszenzsignale aus jedem der Messbereiche in den 121 Arrays gemessen.

Zum Auslesen der Arrays in den einzelnen Probenbehältnissen wird die vorgehend beschriebene Vorrichtung in dem beschriebenen analytischen System eingesetzt und auf maximale Einkopplung des Anregungslichts in den Arrays der ersten 4 der 121 Probenbehältnisse entsprechend der obigen Beschreibung justiert, was mit Position des Filterwechslers für die Anregungswellenlänge kontrolliert wird. Anschliessend wird die Intensität des Fluoreszenzlichts aus den Messbereichen (Spots) der Arrays mit Position des Filterwechslers für die Lumineszenzwellenlänge gemessen. Das Auslesen der Arrays in den weiteren Probenbehältnissen erfolgt sequentiell (für jeweils eine Gruppe von 4 Probenbehältnissen mit Arrays von jeweils 25 diskreten Messbereichen nach der anderen), mittels Translation der Vorrichtung zur nächsten Position für das Auslesen der Lumineszenzsignale aus dem nächsten Probenbehältnis.

## Patentansprüche

1. Vorrichtung, umfassend einen planaren optischen Wellenleiter als Bestandteil einer Sensorplattform und eine mit der Sensorplattform direkt oder über ein dichtendes Medium vermittelt in Kontakt stehende, direkt dicht verschliessende oder über das dichtende Medium dicht verschlossene Schicht (g) mit einer Vielzahl von mindestens zur Seite der Sensorplattform offenen Aussparungen, welche eine Vielzahl von Probenbehältnissen in einer zweidimensionalen Anordnung bilden, wobei jedes Probenbehältnisnur hus einen wellenleitenden Bereich aufweist,
in einem einzelnen Probenbehältnis unterschiedliche biochemische oder biologische Erkennungselemente zur spezifischen Erkennung und Bindung unterschiedlicher Analyten in diskreten Messbereichen (d) immobilisiert sind und diese Messbereiche in optischer Wechselwirkung mit dem Anregungslicht aus dem optischen Wellenleiter, als Bestandteil einer Sensorplattform, stehen, welche
eine Begrenzungsfläche dieser Probenbehältnisses bildet, wobei den Probenbehältnissen zugeführte Proben- oder Reagensflüssigkeiten abgeführt und nachfolgend weitere Proben- oder Reagensflüssigkeiten, gegebenenfalls ohne Waschschritte, den gleichen besagten Probenbehältnissen zugeführt werden können, wobei fünf oder mehr diskrete Messbereiche (d) zweidimensional in dem Probenbehältnis integriert und auf dem wellenleitenden Bereich angeordnet sind.

2. Vorrichtung nach Anspruch 1, **dadurch gekennzeichnet, dass** Messbereiche zur Referenzierung gleicher chemischer oder optischer Parameter in mehreren über die Sensorplattform verteilten Probenbehältnissen verwendet werden, so dass die örtliche Verteilung besagter chemischer oder optischer Parameter auf der Sensorplattform bestimmt werden kann.

3. Vorrichtung nach einem der Ansprüche 1-2, **dadurch gekennzeichnet, dass** der optische Wellenleiter als Bestandteil der Sensorplattform ein optischen Schichtwellenleiter mit einer ersten optisch transparenten Schicht (a) auf einer zweiten optisch transparenten Schicht (b) mit niedrigerem Brechungsindex als Schicht (a) ist.

4. Vorrichtung nach Anspruch 3, **dadurch gekennzeichnet, dass** zur Immobilisierung biologischer oder biochemischer oder synthetischer Erkennungselementen auf der optisch transparenten Schicht (a) eine Haftvermittlungsschicht (f) aufgebracht ist.

5. Vorrichtung nach einem der Ansprüche 3-4, **dadurch gekennzeichnet, dass** die Einkopplung des Anregungslichts zu den Messbereichen (d) mithilfe von einer oder mehreren Gitterstrukturen (c) erfolgt, die in der optisch transparenten Schicht (a) ausgeprägt sind.

6. Vorrichtung nach einem der Ansprüche 3-5, **dadurch gekennzeichnet, dass** Auskopplung von in der optisch transparenten Schicht (a) geführtem Licht mithilfe von Gitterstrukturen (c') erfolgt, die in der optisch transparenten Schicht (a) ausgeprägt sind.

7. Vorrichtung nach einem der Ansprüche 3-6, **dadurch gekennzeichnet, dass** zwischen der optisch transparenten Schicht (a) und den immobilisierten biologischen oder biochemischen oder synthetischen Erkennungselementen eine dünne Metallschicht, vorzugsweise aus Gold oder Silber, gegebenenfalls auf einer zusätzlichen dielektrischen Schicht mit niedrigerem Brechungsindex als der Schicht (a), beispielsweise aus Silica oder Magnesiumfluorid, aufgebracht ist, wobei die Dicke der Metallschicht und der eventuellen weiteren Zwischenschicht so ausgewählt ist, dass ein Oberflächenplasmon bei der Anregungswellenlänge und / oder der Lumineszenzwellenlänge angeregt werden kann.

8. Vorrichtung nach einem der Ansprüche 5-7, **dadurch gekennzeichnet, dass** sich die Gitterstrukturen (c) und gegebenenfalls zusätzlich vorhandenen Gitterstukturen (c') ausserhalb des Bereichs der Probenbehältnisse befinden.

9. Vorrichtung nach einem der Ansprüche 5-7, **dadurch gekennzeichnet dass** sich Gitterstrukturen (c) und gegebenenfalls zusätzlich vorhandene Gitterstrukturen (c') über den Bereich mehrerer oder alle Probenbehältnisse erstrecken.

10. Vorrichtung nach einem der Ansprüche 1-9, **dadurch gekennzeichnet, dass** das Material der mit der Sensorplattform in Kontakt stehenden, dicht verschliessenden Schicht (g) an der Auflageoberfläche zu der Sensorplattform mindestens in der Eindringtiefe des evaneszenten Feldes sowohl für die Anregungsstrahlung als auch für die angeregte Lumineszenzstrahlung transparent ist.

11. Vorrichtung nach Anspruch 10, **dadurch gekennzeichnet, dass** die Schicht (g) als zweilagige Schicht vorliegt, deren erste, welche mit der Oberfläche der Sensorplattform in Kontakt gebracht wird, sowohl für die Anregungsstrahlung als auch für die angeregte Lumineszenzstrahlung transparent ist, während die dann anschliessende, sich im weiteren Abstand von der Sensorplattform befindliche Schicht im Spektralbereich der Anregungsstrahlung und der angeregten Lumineszenzstrahlung absorbierend ist.

12. Vorrichtung nach Anspruch 9, **dadurch gekennzeichnet, dass** das Material der mit der Sensorplattform in Kontakt stehenden, dicht verschliessenden Schicht (g) im Spektralbereich der Anregungsstrahlung und der angeregten Lumineszenzstrahlung absorbierend ist.

13. Vorrichtung nach einem der Ansprüche 1-12, **dadurch gekennzeichnet, dass** das Material der mit der Sensorplattform in Kontakt stehenden, dicht verschliessenden Schicht (g) auf der Sensorplattform selbsthaftend ist.

14. Vorrichtung nach einem der Ansprüche 1-13, **dadurch gekennzeichnet, dass** das Material der mit der Sensorplattform in Kontakt stehenden, dicht verschliessenden Schicht (g) aus einem Polysiloxan besteht.

15. Vorrichtung nach einem der Ansprüche 1-14, **dadurch gekennzeichnet, dass** sich in einem Probenbehältnis 5-1000, bevorzugt 10-400 Messbereiche befinden.

16. Vorrichtung nach einem der Ansprüche 1-15, **dadurch gekennzeichnet, dass** ein einzelner Messbereich in einem Probenbehältnis eine Fläche von 0.001-6 mm² einnimmt, wobei die Messbereiche gleiche oder unterschiedliche Grösse haben.

17. Vorrichtung nach einem der Ansprüche 1-16, **dadurch gekennzeichnet, dass** die Probenbehältnisse jeweils ein Volumen von 100 nl - 1 ml haben.

18. Vorrichtung nach einem der Ansprüche 1-17 **dadurch gekennzeichnet, dass** die Probenbehältnisse auf der von der optisch transparenten Schicht (a) abgewandten Seite, mit Ausnahme von Ein- und / oder Auslassöffnungen für die Zufuhr oder den Auslass der Proben und gegebenenfalls zusätzlicher Reagentien, geschlossen sind und die Zufuhr oder der Auslass von Proben und gegebenenfalls zusätzlicher Reagentien in einem geschlossenen Durchflusssystem erfolgen, wobei im Falle der Flüssigkeitszufuhr zu mehreren Messbereichen oder Segmenten mit gemeinsamen Einlass- und Auslassöffnungen diese bevorzugt spalten- oder zeilenweise adressiert werden.

19. Vorrichtung nach einem der Ansprüche 1-18, **dadurch gekennzeichnet, dass** die Zufuhr der Proben und gegebenenfalls zusätzlichen Reagentien in parallelen oder gekreuzten Mikrokanälen, unter Einwirkung von Druckunterschieden oder elektrischen oder elektromagnetischen Potentialen erfolgt.

20. Vorrichtung nach einem der Ansprüche 1-19 **dadurch gekennzeichnet, dass** die Probenbehältnisse auf der von der optisch transparenten Schicht (a) abgewandten Seite Öffnungen zur lokal adressierten Zugabe oder Entfernung der Proben oder anderer Reagentien besitzen.

21. Vorrichtung nach einem der Ansprüche 1-20, **dadurch gekennzeichnet, dass** Behältnisse für Reagentien vorgesehen sind, welche während des Verfahrens zum Analytnachweis benetzt und mit den Messbereichen in Kontakt gebracht werden.

22. Vorrichtung nach einem der Ansprüche 1-21, **dadurch gekennzeichnet, dass** auf der Sensorplattform optisch oder mechanisch erkennbare Markierungen zur Erleichterung der Justierung in einem optischen System und / oder zur Verbindung mit der die Ausnehmungen für die Probenbehältnisse enthaltenden Schicht (g) aufgebracht sind.

23. Analytisches System zur Bestimmung einer oder mehrerer Lumineszenzen, mit
- mindestens einer Anregungslichtquelle
- einer Vorrichtung nach einem der Ansprüche 1-22
- mindestens einem Detektor zur Erfassung des von dem mindestens einem oder mehreren Messbereichen (d) auf der Sensorplattform ausgehenden Lichts.

24. Analytisches System nach Anspruch 23 mit einer Vorrichtung nach einem der Ansprüche 1-23, **dadurch gekennzeichnet, dass** das von der mindestens einen Lichtquelle ausgesandte Anregungslicht kohärent ist und unter dem Resonanzwinkel zur Einkopplung in die optisch transparente Schicht (a) auf die einen oder mehreren Messbereiche eingestrahlt wird.

25. Analytisches System nach Anspruch 24, **dadurch gekennzeichnet, dass** als Anregungslichtquellen zwei oder mehrere kohärente Lichtquellen mit gleicher oder unterschiedlicher Emissionswellenlänge verwendet werden.

26. Analytisches System nach einem der Ansprüche 23-25, **dadurch gekennzeichnet, dass** zur Detektion mindestens ein ortsauflösender Detektor verwendet wird.

27. Analytisches System nach einem der Ansprüche 23-26, **dadurch gekennzeichnet, dass** zwischen der einen oder mehreren Anregungslichtquellen und der Sensorplattform und /oder zwischen besagter Sensorplattform und dem einen oder mehreren Detektoren optische Komponenten aus der Gruppe verwendet werden, die von Linsen oder Linsensystemen zur Formgestaltung der übertragenen Lichtbündel, planaren oder gekrümmten Spiegeln zur Umlenkung und gegebenenfalls zusätzlich zur Formgestaltung von Lichtbündeln, Prismen zur Umlenkung und gegebenenfalls zur spektralen Aufteilung von Lichtbündeln, dichroischen Spiegeln zur spektral selektiven Umlenkung von Teilen von Lichtbündeln, Neutralfiltern zur Regelung der übertragenen Lichtintensität, optischen Filtern oder Monochromatoren zur spektral selektiven Übertragung von Teilen von Lichtbündeln oder polarisationsselektiven Elementen zur Auswahl diskreter Polarisationsrichtungen des Anregungs- oder Lumineszenzlichts gebildet werden.

28. Analytisches System nach einem der Ansprüche 23-27, **dadurch** gekenzeichnet, dass die Einstrahlung des Anregungslichts auf und Detektion des Emissionslichts von einem oder mehreren Messbereichen sequentiell für einzelne oder mehrere Probenbehältnisse erfolgt.

29. Analytisches System nach Anspruch 28, **dadurch gekennzeichnet, dass** sequentielle Anregung und Detektion unter Verwendung beweglicher optischer Komponenten erfolgt, die aus der Gruppe von Spiegeln, Umlenkprismen und dichroischen Spiegeln gebildet wird.

30. Analytisches System nach Anspruch 29, **dadurch gekennzeichnet, dass** sequentielle Anregung und Detektion unter Verwendung eines im Wesentlichen winkel- und fokusgetreuen Scanners erfolgt.

31. Verfahren zum Lumineszenznachweis eines oder mehrerer Analyten in einer oder mehreren Proben auf mindestens fünf oder mehr, räumlich getrennten Messbereichen mit einer Vorrichtung oder einem analytischen System gemäß einem der vorherigen Ansprüche 1-30 weiter **dadurch gekennzeichnet, dass** den Probenbehältnissen Probenflüssigkeiten zugeführt werden, Anregungslicht in die Messbereiche geleitet wird, hierbei lumineszenzfähige Stoffe in den Proben oder auf den Messbereichen zur Lumineszenz angeregt und die abgestrahlte Lumineszenz gemessen wird.

32. Verfahren nach Anspruch 31 mit einer Vorrichtung nach einem der Ansprüche 1-23, **dadurch gekennzeichnet, dass** (1) die isotrop abgestrahlte Lumineszenz oder (2) in die optisch transparente Schicht (a) eingekoppelte und über die Gitterstruktur (c) ausgekoppelte Lumineszenz oder Lumineszenzen beider Anteile (1) und (2) gleichzeitig gemessen werden.

33. Verfahren nach einem der Ansprüche 31-32, **dadurch gekennzeichnet, dass** zur Erzeugung der Lumineszenz ein Lumineszenzfarbstoff oder lumineszentes Nanopartikel als Lumineszenzlabel verwendet wird, das bei einer Wellenlänge zwischen 300 nm und 1100 nm angeregt werden kann und emittiert.

34. Verfahren nach Anspruch 33. **dadurch gekennzeichnet, dass** das Lumineszenzlabel an den Analyten oder in einem kompetitiven Assay an einen Analogen des Analyten oder in einem mehrstufigen Assay an einen der Bindungspartner der immobilisierten biologischen oder biochemischen oder synthetischen Erkennungselementen oder an die biologischen oder biochemischen oder synthetischen Erkennungselementen gebunden ist.

35. Verfahren nach einem der Ansprüche 33-34, **dadurch gekennzeichnet, dass** ein zweites oder noch weitere Lumineszenzlabel mit gleicher oder unterschiedlicher Anregungswellenlänge wie das erste Lumineszenzlabel und gleicher oder unterschiedlicher Emissionswellenlänge verwendet werden

36. Verfahren nach Anspruch 35, **dadurch gekennzeichnet, dass** das zweite oder noch weitere Lumineszenzlabel bei der gleichen Wellenlänge wie der erste Lumineszenzfarbstoff angeregt werden kann, aber bei anderen Wellenlängen emittieren.

37. Verfahren nach Anspruch 35, **dadurch gekennzeichnet, dass** die Anregungsspektren und Emissionsspektren der eingesetzten Lumineszenzfarbstoffe nur wenig oder gar nicht überlappen.

38. Verfahren nach einem der Ansprüche 31-37, **dadurch gekennzeichnet, dass** neben der Bestimmung einer oder mehrerer Lumineszenzen Änderungen des effektiven Brechungsindex auf den Messbereichen bestimmt werden.

39. Verfahren nach einem der Ansprüche 31-38, **dadurch gekennzeichnet, dass** die einen oder mehreren Lumineszenzen und / oder Bestimmungen von Lichtsignalen bei der Anregungswellenlänge polarisationsselektiv vorgenommen werden.

40. Verfahren nach einem der Ansprüche 39, **dadurch gekennzeichnet, dass** die einen oder mehreren Lumineszenzen bei einer anderen Polarisation als der des Anregungslichts gemessen werden.

41. Verwendung der Vorrichtung nach einem der Ansprüche 1-22 oder des analytischen Systems nach einem der Ansprüche 23-30 zu quantitativen oder qualitativen Analysen zur Bestimmung chemischer, biochemischer oder biologischer Analyten in Screeningverfahren in der Pharmaforschung, der Kombinatorischen Chemie, der Klinischen und Präklinischen Entwicklung, zu Echtzeitbindungsstudien und zur Bestimmung kinetischer Parameter im Affinitätsscreening und in der Forschung, zu qualitativen und quantitativen Analytbestimmungen, insbesondere für die DNA- und RNA-Analytik und die Bestimmung von genomischen oder proteomischen Unterschieden im Genom, wie beispielsweise Einzelnukleotid-Polymorphismen, zur Messung von Protein-DNA-wechselwirkungen, zur Bestimmung von Steuerungsmechanismen für die m-RNA-Expression und für die Protein(bio)synthese, für die Erstellung von Toxizitätsstudien sowie für die Bestimmung von Expressionsprofilen, insbesondere zur Bestimmung von biologischen und chemischen Markerstoffen, wie mRNA, Proteinen, Peptiden oder niedermolekularen organischen (Boten-)Stoffen, sowie zum Nachweis von Antikörpern, Antigenen, Pathogenen oder Bakterien in der pharmazeutischen Produktforschung und - entwicklung, der Human- und Veterinärdiagnostik, der Agrochemischen Produktforschung und -entwicklung, der symptomatischen und präsymptomatischen Pflanzendiagnostik, zur Patientenstratifikation in der pharmazeutischehn Produktentwicklung und für die therapeutische Medikamentenauswahl, zum Nachweis von Pathogenen, Schadstoffen und Erregern, insbesondere von Salmonellen, Prionen, Viren und Bakterien, insbesondere in der Lebensmittel- und Umweltanalytik.

## Claims

1. Device comprising a planar optical waveguide, as part of a sensor platform, and, connected to said platform directly or by means of a sealing medium, a layer (g), which either forms a tight seal directly or is sealed tightly by means of said sealing medium and comprises a multitude of recesses open at least towards the sensor platform, which form a multitude of sample compartments in a two-dimensional arrangement, wherein each sample compartment has only one waveguide region,
in an individual sample compartment different biochemical or biological recognition elements, for the specific recognition and binding of different analytes, are immobilized in discrete measurement areas (d) and said measurement areas are in optical interaction with the excitation light emanating from the optical waveguide, as part of a sensor platform which forms a demarcation of said sample compartments, wherein the sample compartments are able to be cleared from received sample or reagent solutions and to receive, in the following, optionally without washing steps, further sample or reagent solutions, wherein five or more discrete measurement areas (d) are two-dimensionally integrated in the sample compartment and arranged on the waveguide region.

2. Device according to Claim 1, **characterized in that** measurement areas for referencing the same chemical or optical parameters are used in a number of sample compartments distributed over the sensor platform, whereby the local distribution of said chemical or optical parameters on the sensor platform can be determined.

3. Device according to either Claim 1 or 2, **characterized in that** the optical waveguide, as part of the sensor platform, is an optical film waveguide comprising a first optically transparent layer (a) on a second optically transparent layer (b) having a lower refractive index than layer (a).

4. Device according to Claim 3, **characterized in that** an adhesion-promoting layer (f) is deposited to immobilize biological or biochemical or synthetic recognition elements on the optically transparent layer (a).

5. Device according to either Claim 3 or 4, **characterized in that** the excitation light is coupled into the measurement areas (d) using one or more grating structures (c) which are formed in the optically transparent layer (a).

6. Device according to any of Claims 3 to 5, **characterized in that** light guided in the optically transparent layer (a) is coupled out using grating structures (c') which are formed in the optically transparent layer (a).

7. Device according to any of Claims 3 to 6, **characterized in that**, between the optically transparent layer (a) and the immobilized biological or biochemical or synthetic recognition elements, there is deposited a thin metal layer, preferably composed of gold or silver, optionally on an additional dielectric layer having a lower refractive index than the layer (a), composed of silica or magnesium fluoride for example, wherein the thickness of the metal layer and the possible further intermediate layer is selected such that a surface plasmon can be excited at the excitation wavelength and/or the luminescence wavelength.

8. Device according to any of Claims 5 to 7, **characterized in that** the grating structures (c) and any additional grating structures (c') are located outside the region of the sample compartments.

9. Device according to any of Claims 5 to 7, **characterized in that** grating structures (c) and any additional grating structures (c') extend over the region of a number of sample compartments or all sample compartments.

10. Device according to any of Claims 1 to 9, **characterized in that** the material of the tightly sealing layer (g) connected to the sensor platform, at the incumbent surface to the sensor platform, is transparent both for the excitation radiation and for the excited luminescence radiation at least within the penetration depth of the evanescent field.

11. Device according to Claim 10, **characterized in that** the layer (g) is composed of two layers, of which the first layer, which is brought into contact with the surface of the sensor platform, is transparent both for the excitation radiation and for the excited luminescence radiation, whereas the adjacent layer which is located more remotely from the sensor platform is absorbent in the spectral range of the excitation radiation and of the excited luminescence radiation.

12. Device according to Claim 9, **characterized in that** the material of the tightly sealing layer (g) connected to the sensor platform is absorbent in the spectral range of the excitation radiation and of the excited luminescence radiation.

13. Device according to any of Claims 1 to 12, **characterized in that** the material of the tightly sealing layer (g) connected to the sensor platform is self-adhesive on the sensor platform.

14. Device according to any of Claims 1 to 13, **characterized in that** the material of the tightly sealing layer (g) connected to the sensor platform comprises a polysiloxane.

15. Device according to any of Claims 1 to 14, **characterized in that** 5-1000, preferably 10-400, measurement areas are located in one sample compartment.

16. Device according to any of Claims 1 to 15, **characterized in that** an individual measurement area in one sample compartment occupies an area of 0.001-6 mm², wherein the measurement areas are similar or different in size.

17. Device according to any of Claims 1 to 16, **characterized in that** the sample compartments each have a volume of 100 nl to 1 ml.

18. Device according to any of Claims 1 to 17, **characterized in that** the sample compartments are closed at the side facing away from the optically transparent layer (a) except for inlet and/or outlet openings for the supply or removal of the samples and any additional reagents, and the supply or removal of samples and any additional reagents is performed in a closed flow-through system, wherein, when liquid is supplied to multiple measurement areas or segments with common inlet and outlet openings, said inlet and outlet openings are preferably addressed column by column or row by row.

19. Device according to any of Claims 1 to 18, **characterized in that** the supply of the samples and any additional reagents is performed in parallel or crossed microchannels, affected by pressure differences or by electric or electromagnetic potentials.

20. Device according to any of Claims 1 to 19, **characterized in that** the sample compartments have openings for the locally addressed supply or removal of the samples or other reagents at the side facing away from the optically transparent layer (a).

21. Device according to any of Claims 1 to 20, **characterized in that** compartments are provided for reagents, which are wetted and brought into contact with the measurement areas during the method for analyte detection.

22. Device according to any of Claims 1 to 21, **characterized in that** the sensor platform has optically or mechanically recognizable markings provided thereon in order to facilitate the adjustment in an optical system and/or to combine the sensor platform with the layer (g) containing the recesses for the sample compartments.

23. Analytical system for the determination of one or more luminescences, comprising
- at least one excitation light source
- a device according to any of Claims 1 to 22
- at least one detector for recording the light emanating from the at least one or more measurement areas (d) on the sensor platform.

24. Analytical system according to Claim 23 comprising a device according to any of Claims 1 to 23, **characterized in that** the excitation light emitted by the at least one light source is coherent and directed onto the one or more measurement areas at the resonance angle for coupling into the optically transparent layer (a).

25. Analytical system according to Claim 24, **characterized in that** two or more coherent light sources with equal or different emission wavelengths are used as excitation light sources.

26. Analytical system according to any of Claims 23 to 25, **characterized in that** at least one spatially resolving detector is used for detection.

27. Analytical system according to any of Claims 23 to 26, **characterized in that**, between the one or more excitation light sources and the sensor platform and/or between said sensor platform and the one or more detectors, use is made of optical components from the group which comprises lenses or lens systems for the shaping of the transmitted light bundles, planar or curved mirrors for the deviation and optionally additional shaping of light bundles, prisms for the deviation and optionally spectral separation of light bundles, dichroic mirrors for the spectrally selective deviation of parts of light bundles, neutral density filters for the regulation of the transmitted light intensity, optical filters or monochromators for the spectrally selective transmission of parts of light bundles, or polarization-selective elements for the selection of discrete polarization directions of the excitation or luminescence light.

28. Analytical system according to any of Claims 23 to 27, **characterized in that** illumination with the excitation light of and detection of the emission light from one or more measurement areas are performed sequentially for individual or multiple sample compartments.

29. Analytical system according to Claim 28, **characterized in that** sequential excitation and detection is performed using movable optical components which are from the group which comprises mirrors, deviating prisms and dichroic mirrors.

30. Analytical system according to Claim 29, **characterized in that** sequential excitation and detection is performed using an essentially angle- and focus-preserving scanner.

31. Method for the detection of one or more analytes by luminescence in one or more samples on at least five or more spatially separated measurement areas by means of a device or an analytical system according to any of preceding Claims 1 to 30, further **characterized in that** sample solutions are supplied to the sample compartments, excitation light is directed to the measurement areas, thereby exciting substances capable of luminescence in the samples or on the measurement areas to emit luminescence, and the emanated luminescence is measured.

32. Method according to Claim 31 with a device according to any of Claims 1 to 22, **characterized in that** (1) the isotropically emitted luminescence or (2) luminescence that is coupled into the optically transparent layer (a) and outcoupled by the grating structure (c) is measured or luminescences comprising both parts (1) and (2) are measured simultaneously.

33. Method according to either Claim 31 or 32, **characterized in that**, for the generation of the luminescence, a luminescent dye or luminescent nanoparticle is used as a luminescence label, which can be excited and emits at a wavelength between 300 nm and 1100 nm.

34. Method according to Claim 33, **characterized in that** the luminescence label is bound to the analyte or, in a competitive assay, to an analyte analogue or, in a multistep assay, to one of the binding partners of the immobilized biological or biochemical or synthetic recognition elements or to the biological or biochemical or synthetic recognition elements.

35. Method according to either Claim 33 or 34, **characterized in that** a second or more luminescence labels of similar or different excitation wavelengths as the first luminescence label and similar or different emission wavelengths are used.

36. Method according to Claim 35, **characterized in that** the second or more luminescence labels can be excited at the same wavelength as the first luminescent dye, but emit at other wavelengths.

37. Method according to Claim 35, **characterized in that** the excitation and emission spectra of the applied luminescent dyes do not overlap or overlap only partially.

38. Method according to any of Claims 31 to 37, **characterized in that**, besides the determination of one or more luminescences, changes of the effective refractive index on the measurement areas are determined.

39. Method according to any of Claims 31 to 38, **characterized in that** the one or more luminescences and/or determinations of light signals at the excitation wavelength are performed in a polarization-selective manner.

40. Method according to Claim 39, **characterized in that** the one or more luminescences are measured at a polarization that is different from the one of the excitation light.

41. Use of the device according to any of Claims 1 to 22 or of the analytical system according to any of Claims 23 to 30 for quantitative or qualitative analyses for the determination of chemical, biochemical or biological analytes in screening methods in pharmaceutical research, combinatorial chemistry, clinical and preclinical development, for real-time binding studies and for the determination of kinetic parameters in affinity screening and in research, for qualitative and quantitative analyte determinations, especially for DNA and RNA analyses and the determination of genomic or proteomic differences in the genome, such as single nucleotide polymorphisms, for the measurement of protein-DNA interactions, for the determination of control mechanisms for mRNA expression and for protein (bio)synthesis, for the generation of toxicity studies, and for the determination of expression profiles, especially for the determination of biological and chemical marker compounds, such as mRNA, proteins, peptides or low molecular weight organic (messenger) compounds, and for the detection of antibodies, antigens, pathogens or bacteria in pharmaceutical product research and development, human and veterinary diagnostics, agrochemical product research and development, symptomatic and pre-symptomatic plant diagnostics, for patient stratification in pharmaceutical product development and for therapeutic drug selection, for the detection of pathogens, nocuous agents and germs, especially of salmonella, prions, viruses and bacteria, especially in food and environmental analyses.

## Revendications

1. Dispositif comprenant un guide d'onde optique planaire comme constituant d'une plate-forme de capteur et une couche (g) en contact direct ou en contact via un milieu d'étanchéisation avec la plate-forme de capteur, scellant directement de manière étanche ou scellée de manière étanche via le milieu d'étanchéisation, présentant une multitude d'évidements ouverts au moins du côte de la plate-forme, qui forment une multitude de récipients à échantillon dans une disposition bidimensionnelle, chaque récipient d'échantillon présentant une zone guidant les ondes, différents éléments de détection biochimiques ou biologiques pour la détection et la liaison spécifique d'analytes différents étant immobilisés dans des zones de mesure (d) discrètes dans un seul récipient à échantillon et ces zones de mesure étant en interaction optique avec la lumière d'excitation du guide d'onde optique, en tant que constituant d'une plate-forme de capteur, qui forme une surface de délimitation de ce récipient à échantillon, les liquides à échantillon ou de réactif introduits dans les récipients à échantillon pouvant être évacués et ensuite d'autres liquides à échantillon ou à réactif pouvant être introduits, le cas échéant sans étapes de lavage, dans lesdits mêmes récipients à échantillon, cinq zones de mesure (d) discrètes ou plus étant intégrées de manière bidimensionnelle dans le récipient à échantillon et disposées sur la zone guidant les ondes.

2. Dispositif selon la revendication 1, **caractérisé en ce que** les zones de mesure sont utilisées pour référencer des paramètres chimiques ou optiques identiques dans plusieurs récipients à échantillon répartis sur la plate-forme de capteur, de manière telle que la répartition locale desdits paramètres chimiques ou optiques sur la plate-forme de capteur peut être déterminée.

3. Dispositif selon l'une quelconque des revendications 1-2, **caractérisé en ce que** le guide d'onde optique, en tant que constituant de la plate-forme de capteur, est un guide d'onde optique à couches avec une première couche (a) optiquement transparente sur une deuxième couche (b) optiquement transparente présentant un indice de réfraction plus faible que la couche (a).

4. Dispositif selon la revendication 3, **caractérisé en ce qu'**une couche de promoteur d'adhérence (f) est appliquée sur la couche (a) optiquement transparente pour l'immobilisation d'éléments de détection biologiques ou biochimiques ou synthétiques.

5. Dispositif selon l'une quelconque des revendications 3-4, **caractérisé en ce que** le couplage de la lumière d'excitation aux zones de mesure (d) est réalisé à l'aide d'une ou de plusieurs structures de type grille (c) qui sont façonnées dans la couche (a) optiquement transparente.

6. Dispositif selon l'une quelconque des revendications 3-5, **caractérisé en ce que** le découplage de la lumière guidée dans la couche (a) optiquement transparente est réalisé à l'aide de structures de type grille (c') qui sont façonnées dans la couche (a) optiquement transparente.

7. Dispositif selon l'une quelconque des revendications 3-6, **caractérisé en ce qu'**une fine couche en métal, de préférence en or ou en argent, est appliquée entre la couche (a) optiquement transparente et les éléments de détection biologiques ou biochimiques ou synthétiques immobilisés, le cas échéant sur une couche diélectrique supplémentaire présentant un indice de réfraction plus faible que la couche (a), par exemple en silice ou en fluorure de magnésium, l'épaisseur de la couche en métal et de ladite éventuelle autre couche intermédiaire étant choisie de manière telle qu'un plasmon de surface peut être excité à la longueur d'onde d'excitation et/ou à la longueur d'onde de luminescence.

8. Dispositif selon l'une quelconque des revendications 5-7, **caractérisé en ce que** les structures de type grille (c) et les structures de type grille (c') le cas échéant présentes en plus se trouvent en dehors de la zone des récipients à échantillon.

9. Dispositif selon l'une quelconque des revendications 5-7, **caractérisé en ce que** les structures de type grille (c) et les structures de type grille (c') le cas échéant présentes en plus s'étendent sur la zone de plusieurs ou tous les récipients à échantillon.

10. Dispositif selon l'une quelconque des revendications 1-9, **caractérisé en ce que** le matériau de la couche (g) en contact avec la plate-forme de capteur, scellant de manière étanche sur la surface support vers la plate-forme de capteur est transparent au moins dans la profondeur de pénétration du champ évanescent, tant au rayonnement d'excitation qu'au rayonnement de luminescence excité.

11. Dispositif selon la revendication 10, **caractérisé en ce que** la couche (g) se trouve sous forme de couche à deux strates, dont la première, qui est mise en contact avec la surface de la plate-forme de capteur, est transparente au rayonnement d'excitation ainsi qu'au rayonnement de luminescence excité, alors que la couche consécutive, se trouvant à une plus grande distance de la plate-forme de capteur, est absorbante dans la plage spectrale du rayonnement d'excitation et du rayonnement de luminescence excité.

12. Dispositif selon la revendication 9, **caractérisé en ce que** le matériau de la couche (g) en contact avec la plate-forme de capteur, scellant de manière étanche est absorbante dans la plage spectrale du rayonnement d'excitation et du rayonnement de luminescence excité.

13. Dispositif selon l'une quelconque des revendications 1-12, **caractérisé en ce que** le matériau de la couche (g) en contact avec la plate-forme de capteur, scellant de manière étanche est autoadhésive sur la plate-forme de capteur.

14. Dispositif selon l'une quelconque des revendications 1-13, **caractérisé en ce que** le matériau de la couche (g) en contact avec la plate-forme de capteur, scellant de manière étanche est constitué par un polysiloxane.

15. Dispositif selon l'une quelconque des revendications 1-14, **caractérisé en ce qu'**un récipient à échantillon contient 5-1000, de préférence 10-400 zones de mesure.

16. Dispositif selon l'une quelconque des revendications 1-15, **caractérisé en ce qu'**une seule zone de mesure dans un récipient à échantillon occupe une surface de 0,001-6 mm², les zones de mesure présentant des tailles identiques ou différentes.

17. Dispositif selon l'une quelconque des revendications 1-16, **caractérisé en ce que** les récipients à échantillon présentent à chaque fois un volume de 100 n1-1 ml.

18. Dispositif selon l'une quelconque des revendications 1-17, **caractérisé en ce que** les récipients à échantillon, sur le côté opposé à la couche (a) optiquement transparente, à l'exception d'ouvertures d'admission et/ou d'évacuation pour l'alimentation ou l'évacuation des échantillons et le cas échéant de réactifs supplémentaires, sont fermés et l'alimentation ou l'évacuation d'échantillons et le cas échéant de réactifs supplémentaires ont lieu dans un système d'écoulement fermé, où, dans le cas de l'alimentation de liquide vers plusieurs zones de mesure ou segments avec des ouvertures d'alimentation et d'évacuation communes, ceux-ci sont de préférence adressés en colonnes en lignes.

19. Dispositif selon l'une quelconque des revendications 1-18, **caractérisé en ce que** l'alimentation des échantillons et le cas échéant de réactifs supplémentaires a lieu dans des microcanaux parallèles ou croisés, sous l'effet de différences de pression ou de potentiels électriques ou électromagnétiques.

20. Dispositif selon l'une quelconque des revendications 1-19, **caractérisé en ce que** les récipients à échantillon présentent, sur le côté opposé à la couche (a) optiquement transparente, des ouvertures pour l'addition ou l'élimination adressée localement des échantillons ou d'autres réactifs.

21. Dispositif selon l'une quelconque des revendications 1-20, **caractérisé en ce que** les récipients sont prévus pour des réactifs qui sont mouillés pendant le procédé de détection d'analytes et mis en contact avec les zones de mesure.

22. Dispositif selon l'une quelconque des revendications 1-21, **caractérisé en ce que** des marquages optiquement ou mécaniquement détectables sont appliqués sur la plate-forme de capteur pour faciliter l'ajustement dans un système optique et/ou pour un raccordement avec la couche (g) contenant les évidements pour les récipients à échantillon.

23. Système analytique destiné à la détermination d'une ou de plusieurs luminescences, présentant
- au moins une source de lumière d'excitation
- un dispositif selon l'une quelconque des revendications 1-22,
- au moins un détecteur pour capter la lumière partant de ladite au moins une ou desdites plusieurs zones de mesure (d) sur la plate-forme de capteur.

24. Système analytique selon la revendication 23 présentant un dispositif selon l'une quelconque des revendications 1-23, **caractérisé en ce que** la lumière d'excitation partant de ladite au moins une source de lumière est cohérente et est irradiée sur ladite une ou lesdites plusieurs zones de mesure sous l'angle de résonance pour le couplage dans la couche (a) optiquement transparente.

25. Système analytique selon la revendication 24, **caractérisé en ce qu'**on utilise comme sources lumineuses d'excitation deux sources de lumière cohérente ou plus présentant une longueur d'onde d'émission identique ou différente.

26. Système analytique selon l'une quelconque des revendications 23-25, **caractérisé en ce qu'**on utilise pour la détection au moins un détecteur à résolution spatiale.

27. Système analytique selon l'une quelconque des revendications 23-26, **caractérisé en ce qu'**on utilise entre ladite une ou lesdites plusieurs sources de lumière d'excitation et la plate-forme de capteur et/ou entre ladite plate-forme de capteur et ledit un ou lesdits plusieurs détecteurs des composants optiques du groupe formé par les lentilles ou les systèmes de lentilles pour la conception des formes du faisceau lumineux transmis, les miroirs planaires ou courbés pour dévier et le cas échéant en plus pour la conception des formes des faisceaux lumineux, les prismes pour dévier et le cas échéant la répartition spectrale de faisceaux lumineux, les miroirs dichroïques pour la déviation spectralement sélective de parties de faisceaux lumineux, les filtres neutres pour le réglage de l'intensité lumineuse transmise, les filtres optiques ou les monochromateurs pour la transmission spectralement sélective de parties de faisceaux lumineux ou les éléments sélectifs de polarisation pour le choix d'orientations de polarisation discrètes de la lumière d'excitation ou de luminescence.

28. Système analytique selon l'une quelconque des revendications 23-27, **caractérisé en ce que** l'irradiation de la lumière d'excitation sur et la détection de la lumière d'émission d'une ou de plusieurs zones de mesure sont réalisées séquentiellement pour un ou plusieurs récipients d'échantillon.

29. Système analytique selon la revendication 28, **caractérisé en ce que** l'excitation et la détection séquentielles sont réalisées en utilisant des composants optiques mobiles, formés par le groupe des miroirs, des prismes de déviation et des miroirs dichroïques.

30. Système analytique selon la revendication 29, **caractérisé en ce que** l'excitation et la détection séquentielles sont réalisées en utilisant un scanner préservant essentiellement l'angle et la focalisation.

31. Procédé pour la détection de la luminescence d'un ou de plusieurs analytes dans un ou plusieurs échantillons sur au moins cinq zones de mesure ou plus, séparées dans l'espace, avec un dispositif ou un système analytique selon l'une quelconque des revendications précédentes 1-30, en outre **caractérisé en ce que** des liquides d'échantillon sont introduits dans les récipients à échantillon, une lumière d'excitation est guidée dans les zones de mesure, les substances aptes à une luminescence dans les échantillons ou sur les zones de mesure sont excitées en luminescence et la luminescence émise est mesurée.

32. Procédé selon la revendication 31 présentant un dispositif selon l'une quelconque des revendications 1-22, **caractérisé en ce que** (1) la luminescence émise de manière isotrope ou (2) la luminescence couplée dans la couche (a) optiquement transparente et découplée via la structure de type grille (c) ou les luminescences des deux proportions (1) et (2) sont mesurées simultanément.

33. Procédé selon l'une quelconque des revendications 31-32, **caractérisé en ce qu'**on utilise, pour la production de la luminescence, un colorant luminescent ou des nanoparticules luminescentes comme marqueur de luminescence, qui peut être excité et qui émet à une longueur d'onde entre 300 nm et 1100 nm.

34. Procédé selon la revendication 33, **caractérisé en ce que** le marqueur de luminescence est lié aux analytes ou, dans un essai compétitif, à un analogue de l'analyte ou, dans un essai à plusieurs étapes, à un des partenaires de liaison des éléments de détection biologiques ou biochimiques ou synthétiques immobilisés ou aux éléments de détection biologiques ou biochimiques ou synthétiques.

35. Procédé selon l'une quelconque des revendications 33-34, **caractérisé en ce qu'**on utilise un deuxième marqueur de luminescence ou des marqueurs de luminescence supplémentaires présentant une longueur d'onde d'excitation identique à ou différente du premier marqueur de luminescence et une longueur d'onde d'émission identique ou différente.

36. Procédé selon la revendication 35, **caractérisé en ce que** le deuxième marqueur de luminescence ou encore d'autres marqueurs de luminescence peuvent être excités à la même longueur d'onde que le premier colorant de luminescence, mais émettent à d'autres longueurs d'onde.

37. Procédé selon la revendication 35, **caractérisé en ce que** les spectres d'excitation et d'émission des colorants de luminescence utilisés ne se recouvrent que peu, voire pas du tout.

38. Procédé selon l'une quelconque des revendications 31-37, **caractérisé en ce qu'**en plus de la détermination d'une ou de plusieurs luminescences, on détermine des modifications de l'indice de réfraction effectif sur les zones de mesure.

39. Procédé selon l'une quelconque des revendications 31-38, **caractérisé en ce que** ladite une ou lesdites plusieurs luminescences et/ou déterminations de signaux lumineux à la longueur d'onde d'excitation sont réalisées de manière sélective en ce qui concerne la polarisation.

40. Procédé selon la revendication 39, **caractérisé en ce que** ladite une ou lesdites plusieurs luminescences sont mesurées à une autre polarisation que celle de la lumière d'excitation.

41. Utilisation du dispositif selon l'une quelconque des revendications 1-22 ou du système analytique selon l'une quelconque des revendications 23-30 pour des analyses quantitatives ou qualitatives en vue de la détermination d'analytes chimiques, biochimiques ou biologiques dans des procédés de criblage dans la recherche pharmaceutique, la chimie combinatoire, le développement clinique et préclinique, pour des études de liaison en temps réel et pour la détermination de paramètres cinétiques dans le criblage d'affinité et dans la recherche, pour des déterminations qualitatives et quantitatives d'analytes, en particulier pour l'analyse d'ADN et d'ARN et la détermination de différences génomiques ou protéomiques dans le génome, telles que par exemple les polymorphismes de nucléotides individuels, pour la mesure d'interactions protéine-ADN, pour la détermination de mécanismes de contrôle pour l'expression d'ARNm et pour la (bio)synthèse de protéines, pour la réalisation d'études de toxicité ainsi que pour la détermination de profils d'expression, en particulier pour la détermination de substances de marquage biologiques et chimiques, telles que l'ARNm, les protéines, les peptides ou les substances (messagères) organiques de bas poids moléculaire, ainsi que pour la détection d'anticorps, d'antigènes, d'agents pathogènes ou de bactéries dans la recherche et le développement de produits pharmaceutiques, le diagnostic humain et vétérinaire, la recherche et le développement de produits agrochimiques, le diagnostic végétal symptomatique et présymptomatique, pour la stratification de patients dans le développement de produits pharmaceutiques et pour le choix thérapeutique de médicaments, pour la détection d'agents pathogènes, de substances nuisibles et de parasites, en particulier de salmonelles, de prions, de virus et de bactéries, en particulier dans l'analyse alimentaire et environnementale.
